# EUROPEAN PATENT SPECIFICATION

(11) **EP 3 295 886 B1**
(45) Date of publication and mention of the grant of the patent: **20.11.2019**
(21) Application number: 17175335.3
(22) Date of filing: 31.07.2012
(51) Int. Cl.: A61B 18/18, A61B 18/00, A61N 5/02, A61N 5/04

(54) **APPLICATOR AND TISSUE INTERFACE MODULE FOR DERMATOLOGICAL DEVICE**
APPLIKATOR UND GEWEBESCHNITTSTELLENMODUL FÜR EINE DERMATOLOGISCHE VORRICHTUNG
APPLICATEUR ET MODULE D'INTERFACE DE TISSU POUR DISPOSITIF DERMATOLOGIQUE

(30) Priority: 01.08.2011 US 201161513834 P; 03.11.2011 US 201161555410 P; 19.07.2012 US 201261673697 P
(43) Date of publication of application: 21.03.2018
(62) Divisional of application: 12819525.2
(73) Proprietor: miraDry, Inc., Santa Clara, CA 95051 (US)
(72) Inventor: BEN-HAIM, Yoav, Sunnyvale, California 94085 (US); BENTLEY, Peter J., Sunnyvale, California 94085 (US); CHUN, Dong Hoon, Sunnyvale, California 94085 (US); FRANCIS, Daniel E., Sunnyvale, California 94085 (US); JOHNSON, Jessi E., Sunnyvale, California 94085 (US); SHAN, Kevin, Sunnyvale, California 94085 (US); SU, Ted Y., Sunnyvale, California 94085 (US); KIM, Steven W., Sunnyvale, California 94085 (US)
(74) Representative: Murray, David Craig

(56) References cited:
- WO-A1-2010/047818
- JP-A- 2006 503 618
- US-A1- 2010 268 220

## Description

### FIELD

This disclosure relates generally to application of energy to tissue. More specifically, this disclosure relates to application of energy to tissue to treat conditions of the skin, epidermis, dermis and hypodermis.

### BACKGROUND

Hyperhidrosis or excessive sweating is a common disorder and can result in excessive underarm, facial, or foot sweating. Excessive sweating can cause both physical side-effects, including dehydration and infections, as well as emotional side-effects such as embarrassment.

Many forms of treatment of hyperhidrosis are currently known, including medications, antiperspirants, botox, and ablation therapy.

### SUMMARY OF THE DISCLOSURE

The invention is as defined in the appended claims.
A tissue interface module for use with an applicator in a microwave-based tissue modification system is provided, the tissue interface module comprising an attachment mechanism on a proximal side of the tissue interface module adapted to attach to an applicator, an applicator chamber adapted to receive a microwave antenna, a cooling element, and a vacuum port of the applicator, the applicator chamber comprising a bio-barrier on a distal side, a tissue acquisition chamber having a tissue acquisition opening on a distal side of the tissue interface module, and a filter disposed between, and communicating with, the applicator chamber and the tissue acquisition chamber, the filter comprising openings configured to permit air to pass and to prevent liquid from passing.

In some embodiments, the tissue interface module further comprises a variable flow restrictor between, and in communication with, the tissue acquisition chamber and the filter. In one embodiment, the variable flow restrictor comprises a flexible element adapted to expand a flow opening between the tissue acquisition chamber and the filter in response to a pressure difference between the tissue acquisition chamber and the filter.

In various embodiments, the attachment mechanism comprises a magnetic element adapted to magnetically attach to a corresponding element in the applicator.

In some embodiments, the tissue interface module further comprises a tissue interface module engagement surface adapted to engage with a corresponding applicator engagement surface on the applicator, the tissue interface module engagement surface being disposed at an angle of approximately 22.5 degrees with respect to the bio-barrier. In other embodiments, the tissue interface module further comprises a tissue interface module engagement surface adapted to engage with a corresponding applicator engagement surface on the applicator, the tissue interface module engagement surface being disposed at an angle of approximately 17.5 degrees to 27.5 degrees with respect to the bio-barrier. In additional embodiments, the tissue interface module further comprises a tissue interface module engagement surface adapted to engage with a corresponding applicator engagement surface on the applicator, the tissue interface module engagement surface being disposed at an angle of approximately 12.5 degrees to 32.5 degrees with respect to the bio-barrier.

In some embodiments, the tissue interface module comprises a vacuum flow path from the tissue acquisition chamber, through the filter, into the applicator chamber. In other embodiments, the tissue interface module comprises a vacuum flow path from the tissue acquisition chamber, through the filter, through the applicator chamber, into the vacuum port of the applicator.

In some embodiments, the tissue interface module further comprises a second filter disposed between, and communicating with, the applicator chamber and the tissue acquisition chamber, the second filter comprising openings configured to permit air to pass and to prevent liquid from passing. In one embodiment, the filter and the second filter are positioned on opposing sides of the bio-barrier. In other embodiments, the bio-barrier comprises approximately the same surface area as the filter and the second filter combined.

In one embodiment, the attachment mechanism comprises a ferromagnetic plate adapted to attach to the applicator upon completion of a magnetic circuit.

A method of treating tissue of a patient is also provided, comprising attaching a tissue interface module to an applicator to place a microwave antenna, a cooling plate, and a vacuum port within an applicator chamber of the tissue interface module, placing a distal opening of a tissue acquisition chamber of the tissue interface module against a tissue surface, drawing a vacuum from a vacuum source in the applicator through the applicator chamber, a filter between the applicator chamber and the tissue acquisition chamber, and applying microwave energy to the patient's tissue.

In some embodiments, the method further comprises varying a size of an opening between the tissue acquisition chamber and the filter during the step of drawing a vacuum. In some embodiments, the varying step comprises moving a flexible member to change the size of the opening.

In some embodiments, the attaching step comprises magnetically coupling the tissue interface module to the applicator.

In some embodiments, the applicator chamber comprises a bio-barrier on a distal side, the attaching step comprising engaging a magnetic tissue interface module engagement surface with a corresponding applicator engagement surface on the applicator, the tissue interface module engagement surface being disposed at an angle of approximately 17.5 degrees to 27.5 degrees with respect to the bio-barrier.

A microwave-based tissue modification system is provided, comprising a microwave applicator comprising a microwave antenna, a cooling element, and a vacuum port, and a tissue interface module comprising: an attachment mechanism on a proximal side of the tissue interface module adapted to attach to the microwave applicator; an applicator chamber adapted to connect to the microwave antenna, the cooling element, and the vacuum port of the microwave applicator, the applicator chamber comprising a bio-barrier on a distal side, a tissue acquisition chamber having a tissue acquisition opening on a distal side of the tissue interface module, and a filter disposed between, and communicating with, the applicator chamber and the tissue acquisition chamber, the filter comprising openings configured to permit air to pass and to prevent liquid from passing.

In some embodiments, the tissue modification system further comprises a variable flow restrictor between, and in communication with, the tissue acquisition chamber and the filter.

In other embodiments, the attachment mechanism comprises a magnetic element adapted to magnetically attach to a corresponding element in the applicator.

In additional embodiments, the tissue modification system further comprises a tissue interface module engagement surface adapted to engage with a corresponding applicator engagement surface on the microwave applicator, the tissue interface module engagement surface being disposed at an angle of approximately 17.5 degrees to 27.5 degrees with respect to the bio-barrier.

In some embodiments, the tissue modification system further comprises a vacuum flow path from the tissue acquisition chamber, through the filter, through the applicator chamber, into the vacuum port of the microwave applicator.

In another embodiment, the tissue interface module further comprises a second filter disposed between, and communicating with, the applicator chamber and the tissue acquisition chamber, the second filter comprising openings configured to permit air to pass and to prevent liquid from passing. In some embodiments, the filter and the second filter are positioned on opposing sides of the bio-barrier.

A tissue interface module for use with an applicator in a microwave-based tissue modification system is also provided, the tissue interface module comprising an attachment mechanism on a proximal side of the tissue interface module adapted to attach to an applicator, the attachment mechanism comprising an engagement surface that forms an angle of approximately 17.5 degrees to 27.5 degrees from horizontal, an applicator chamber adapted to connect to a microwave antenna, a cooling element, and a vacuum port of the applicator, the applicator chamber comprising a bio-barrier on a distal side, wherein the bio-barrier is configured to prevent air and liquid from passing, a tissue acquisition chamber having a tissue acquisition opening defined by a skirt on a distal side of the tissue interface module; and a filter disposed between, and communicating with, the applicator chamber and the tissue acquisition chamber, the filter comprising openings configured to permit air to pass and to prevent liquid from passing.

In additional embodiments, the tissue interface module further comprises a vacuum flow path from the tissue acquisition chamber, through the filter, through the applicator chamber, into the vacuum port of the microwave applicator.

In other embodiments, the tissue interface module further comprises a second filter disposed between, and communicating with, the applicator chamber and the tissue acquisition chamber, the second filter comprising openings configured to permit air to pass and to prevent liquid from passing. In some embodiments, the filter and the second filter are positioned on opposing sides of the bio-barrier.

In some embodiments, the tissue interface module further comprises a fluid trap disposed between the tissue acquisition chamber and the filter, the fluid trap configured to capture tissue and liquid.

A method of treating tissue of a patient is provided, comprising mating a tissue interface module to an applicator to place a microwave antenna, a cooling plate, and a vacuum port within an applicator chamber of the tissue interface module, actuating a magnet to complete a magnetic circuit between an attachment mechanism of the tissue interface module and the applicator, placing a distal opening of a tissue acquisition chamber of the tissue interface module against a tissue surface, drawing a vacuum from a vacuum source in the applicator through the applicator chamber, a filter between the applicator chamber and the tissue acquisition chamber, and applying microwave energy to the patient's tissue.

A consumable medical device is also provided, said medical device comprising an applicator chamber, said applicator chamber being positioned on a proximal side of said consumable, a tissue chamber, said tissue chamber being positioned on a distal side of said consumable medical device, a first bio-barrier positioned between said applicator chamber and said tissue chamber, said first bio-barrier being substantially impermeable, flexible, microwave transparent, a vacuum path, said vacuum path extending from a distal end of said applicator chamber to a proximal end of said tissue chamber and comprising, a second bio-barrier, a vacuum trap, an expandable aperture, being adapted to facilitate the flow of air from said tissue chamber, through said expandable aperture, through said vacuum trap, through said second bio-barrier and into said applicator chamber.

In some embodiments, the consumable further comprises a shell, an insert, said insert being positioned in said shell to form a body of said consumable, a gasket, said gasket being positioned on said insert, providing a vacuum seal between said insert and said shell on a distal side of said gasket, being shaped to provide a vacuum seal to an applicator on a proximal side of said gasket, forming a portion of said vacuum trap.

In some embodiments, the consumable further comprises a reflector, said reflector reflecting at least a portion of any microwave energy entering said applicator chamber and being electrically isolated from an applicator positioned in said applicator chamber, being positioned between said shell and said insert having a distal end surrounding at least a portion of said tissue chamber.

In one embodiment, the consumable further comprises a latch plate, said latch plate being positioned in said applicator chamber, being positioned on said insert, and forming a predetermined angle with said first bio-barrier when said first bio-barrier is in a first position.

A method of pulling air through a consumable medical device is provided, said method comprising the steps of creating a vacuum in an applicator chamber of said consumable medical device, said applicator chamber being separated from a tissue chamber by a first bio-barrier, said first bio-barrier being flexible and impermeable to bodily fluids and air, pulling air into said applicator chamber from a vacuum trap through a second bio-barrier, said second bio-barrier being permeable to air but substantially impermeable to bodily fluids, pulling air into said vacuum trap through an expandable aperture, wherein said expandable aperture substantially surrounds said tissue chamber, is formed at least in part by said first bio-barrier, opens upon the application of vacuum to said applicator chamber, which pulls said first bio-barrier into said applicator chamber (against a cooling plate), creating a vacuum in said tissue chamber, and pulling tissue positioned outside said tissue chamber into said tissue chamber using said vacuum created in said tissue chamber.

A method of transmitting energy to a patient for the purpose of reducing sweat is provided, the method comprising the steps of Transmitting the energy, through an applicator comprising, An antenna, A Field spreader, A Fluid channel, and A Cooling plate, Transmitting the energy through a consumable comprising An applicator chamber, a Flexible bio-barrier, and A Tissue chamber.

A consumable including a Flexible bio-barrier and cooling plate configured to cooperate to form expandable channel connecting a tissue chamber to an applicator chamber, said consumable including a vacuum path wherein air from a the tissue chamber passes through The Expandable channel, a Fluid trap, a Second bio-barrier, vacuum channels separating second bio-barrier from an attachment mechanism, and An Applicator chamber.

In some embodiments, the attachment mechanism comprises a magnetic plate.

Another embodiment comprises a multifunctional connector adapted to connect an applicator to a microwave generator console through a cable assembly, said connector comprising: a cooling fluid connector, a cooling fluid return connector, a microwave connector, electronic connectors, and vacuum connectors.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
Fig. 1 illustrates a physician with an applicator and a patient.
Fig. 2 shows a perspective view of a tissue interface module attached to an applicator.
Fig. 3 illustrates a perspective view of a tissue interface module detached from an applicator.
Fig. 4 shows an end view of a multifunction connector.
Fig. 5 illustrates an end view of a tissue interface module.
Fig. 6 is a top view of a tissue interface module.
Fig. 7 shows a top perspective view of a tissue interface module.
Fig. 8 illustrates a top perspective view of an alternate tissue interface module.
Fig. 9 shows an exploded top perspective view of a tissue interface module.
Fig. 10 is an exploded top perspective view of an alternate tissue interface module.
Fig. 11 shows a side cutaway view of a tissue interface module.
Fig. 12 illustrates a side cutaway perspective view of a tissue interface module.
Fig. 13 is a perspective end view of an insert assembly from a tissue interface module.
Fig. 14 is an exploded perspective side view of the insert assembly of Fig. 13.
Fig. 15 shows an end view of an applicator without a tissue interface module.
Fig. 16 illustrates a cutaway view of a section of an applicator and a portion of tissue interface module.
Figs. 17A-17B re a side cutaway views of a portion of an applicator and a portion of tissue interface module.
Fig. 18 illustrates a side cutaway view of a section of an applicator and a tissue interface module with tissue engaged and vacuum applied.
Fig. 19 shows a side cutaway view of a section of an applicator and a tissue interface module showing an air path with vacuum applied.
Fig. 20 is a side cutaway perspective view of an applicator and a tissue interface module showing internal components of the applicator, including vacuum conduits.
Fig. 21 illustrates a side cutaway perspective view of an applicator showing internal components of the applicator.
Fig. 22 shows a side cutaway perspective view of an applicator with a tissue interface module attached to the applicator and showing a portion of magnetic drive components.
Fig. 23 shows a side cutaway view of a tissue interface module.
Fig. 24 illustrates a side cutaway perspective view of a tissue interface module.
Fig. 25 illustrates a end cutaway view of a section of an applicator and a portion of a tissue interface module.
Fig. 26 is a side cutaway view of a portion of the applicator and a portion of a tissue interface module.
Fig. 27 illustrates a side cutaway view of a section of an applicator and a tissue interface module as tissue is pulled into a tissue acquisition chamber by applied vacuum.
Fig. 28 shows a side cutaway view of a section of an applicator and a tissue interface module showing air paths with vacuum applied.
Fig. 29 shows a side cutaway view of a tissue interface module of another embodiment of the invention.
Fig. 30 shows a side cutaway view of a section of an applicator and a tissue interface module.
Fig. 31 is a side cutaway view of a portion of an applicator and a portion of a tissue interface module.
Fig. 32 shows a side cutaway view of a section of an applicator and a tissue interface module showing an air path with vacuum applied.

### DETAILED DESCRIPTION

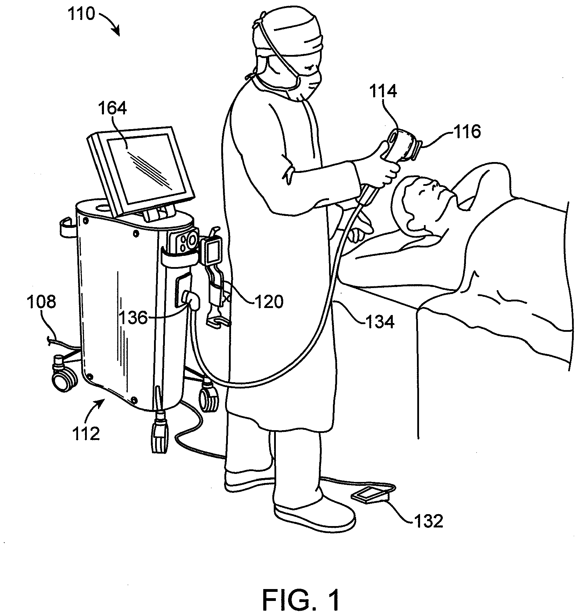

Fig. 1 illustrates a Physician treating a patient with energy delivery system 110. The energy delivery system 110 can include a console 112, applicator 114 and tissue interface module 116. Console 112 may include a display 164, power cord 108, holster 120 and foot pedal switch 132. Display 164 may be used to show a graphical user interface to guide the physician through the treatment steps, such graphical user interface may include a color map of treatment temperatures, a placement count indicator and a placement positioning arrow, for example. Applicator 114 can include cable assembly 134 and multifunction connector 136. The energy delivery system can be configured to deliver energy to tissue of the patient. In some embodiments, the energy delivery system is configured to deliver microwave energy to the skin of the patient to treat a condition of the skin, such as hyperhidrosis, excessive sweating, bromhidrosis, cellulite, fat, wrinkles, acne or unwanted hair.

When the system 110 is assembled, the applicator 114 (which may also be referred to as a hand piece) can be connected to the console 112 (which may also be referred to as a generator) via multifunction connector 136. The console can be configured to generate energy (e.g. microwave energy) at a frequency of, for example, 5.8 gigahertz. In some embodiments, the applicator can be connected to the console with a microwave cable, a tensile cord, a USB cable and vacuum tubing, for example. The applicator may also be connected to a tissue interface module 116 (which may also be referred to as a consumable, disposable, tissue interface, applicator-tissue interface or biotip). A foot pedal switch may be connected to the generator to control the console, or alternatively, switches or buttons on the applicator itself can control the console.

In some embodiments, the console also includes a vacuum source, a cooling fluid source, (e.g., a chiller), a cooling fluid pump, an amplifier, a microwave generator, and control circuitry (not shown). These features of the console are used to generate the vacuum pressure, cooling fluid and microwave energy which may be transmitted through multifunction connector 136 and cable assembly 134 to applicator 114.

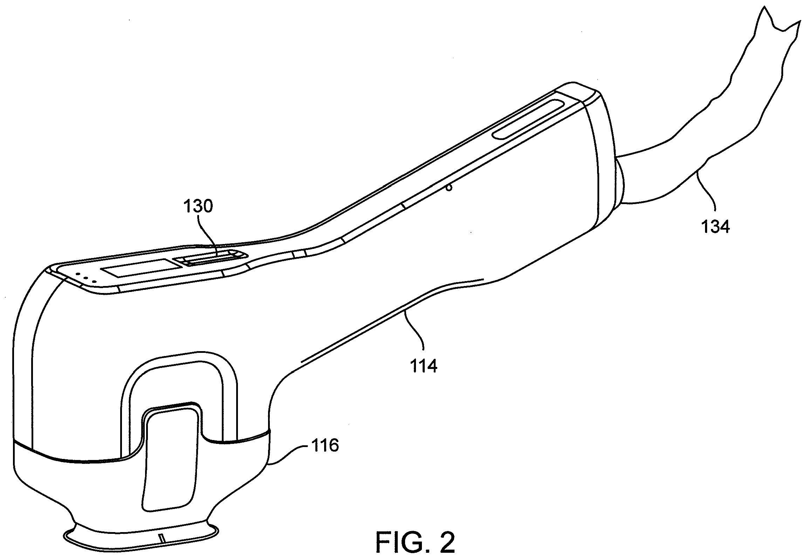

Fig. 2 shows a perspective view of applicator 114 with the tissue interface module 116 attached to the applicator. Cable assembly 134 is shown extending from a proximal portion of the applicator. Applicator switch 130 can be disposed on the handle and can be used to initiate treatment from the applicator. The applicator can also include main control circuitry adapted to control LED indicators, an antenna switch, and applicator switch 130. In some embodiments, the main control circuitry can be designed to receive signals indicative of the direct or reflected power measured at each antenna in the applicator.

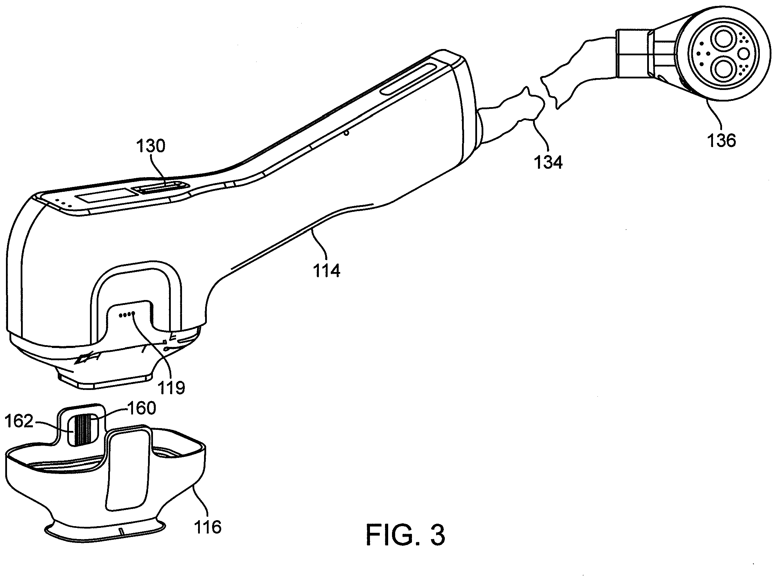

Fig. 3 shows a perspective view of applicator 114 with the tissue interface module 116 detached from the applicator. Removal of the tissue interface module reveals electrical contacts 119, which are configured to engage electrodes 160 and printed circuit board 162 positioned on one or both sides of the tissue interface module. Electrodes 160 may be used to, for example, detect proper alignment of the tissue interface module when attached to the applicator. A security chip on the printed circuit board may also be included, along with ESD protection such as, for example, an ESD capacitor. An integrated circuit (not shown in this figure) can also be included to, for example, detect proper alignment. In some embodiments, the printed circuit board and integrated circuit can detect re-use of a previously used tissue interface module. Such information may be used to, for example, notify the user that a new tissue interface module should be used or prevent the system from re-using the tissue interface module, which may be contaminated with, for example, biological fluids from a previous patient. Fig. 3 also shows an end view of a multifunction connector 136 disposed at the proximal end of cable assembly 134 for attachment of the applicator to the console of Fig. 1.

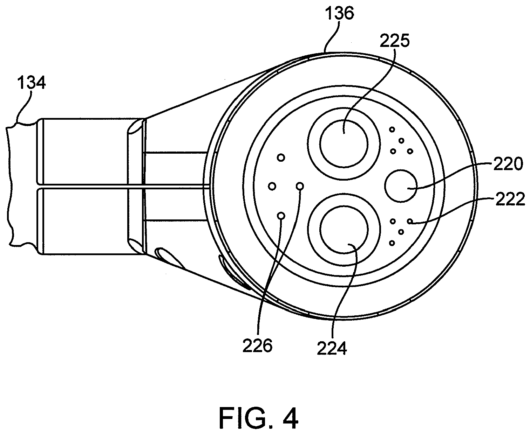

Fig. 4 shows an end view of multifunction connector 136 and cable assembly 134. In Fig. 4, multifunction connector 136 includes cooling fluid connector 224, cooling fluid return connector 225, microwave connector 220, electronic connectors 222 and vacuum connectors 226. The multifunction connector 136 and cable assembly 134 provide a simple connection between the console 112 of Fig. 1 and the applicator 114 of Figs. 2-3, allowing the applicator to receive the microwave energy, electrical energy, cooling fluid, and vacuum necessary for treatment procedures.

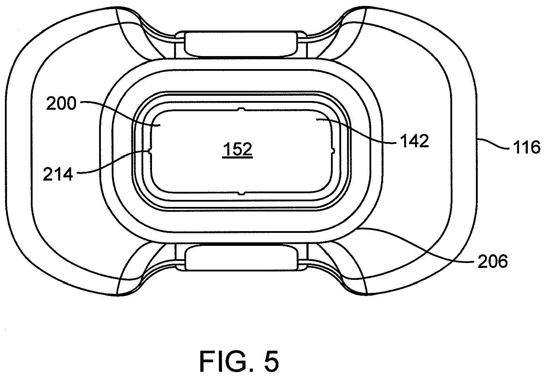

Fig. 5 illustrates an end view of tissue interface module 116 as viewed from the side of the module that contacts tissue. The tissue interface module 116 can include a tissue acquisition chamber 142 having a tissue interface surface 200, a first bio-barrier 152, vacuum notches 214, and skirt 206. The tissue acquisition chamber can be sized to facilitate tissue acquisition in the treatment region of the patient. The tissue acquisition chamber can be sized to prevent interference with energy radiated from the applicator. In some embodiments, the tissue acquisition chamber can be sized to be approximately 1.54 inches long by 0.7 inches wide, having a depth of approximately .255 - .295 inches. The tissue acquisition chamber may include corners having a radius of approximately .1875 inches at a distal end thereof. The tissue acquisition chamber is used to properly position tissue in the interface module and adjacent the distal end of the applicator.

The skirt 206 may be made from, for example, a compliant medical grade plastic (thermal palastic elastomer (TPE)) such as, for example, urethane, silicone, natural or synthetic rubber, elastomeric material, urethane foam with silicone, compliant plastic or a rubber seal coating. A suitable skirt may have a height of between 0.15" and 0.40" and more specifically, approximately 0.25" above the tissue acquisition chamber when the skirt is not compressed. In some embodiments, the skirt can have a durometer density rating (softness) of approximately 60A, or between 40A and 60A, or between 20A and 80A. In one embodiment, the skirt 206 may include inner walls having an average angle of approximately 53 degrees when not compressed. In some embodiments, the skirt 206 may be clear or see-through to assist the physician in properly positioning the applicator with the tissue to be treated, by, for example, aligning the skirt with temporary markings on the patient's skin.

The first bio-barrier 152, which may also be referred to as a membrane or first membrane, is configured to be substantially impermeable to both liquids (e.g. bodily fluids such as blood or sweat) and gas (e.g. air). In some embodiments, the first bio-barrier may be constructed of impermeable materials, such as, for example, polyurethane film and may have a thickness of, for example, 0.0005 inches or 0.00085 inches. In other embodiments, the first bio-barrier may have a thickness of between approximately 0.00075 inches and 0.001 inches. Bio-barrier 152 is further designed to be sufficiently flexible to conform to the tissue treatment surface 502 of applicator 114 without creating bubbles or voids. In some embodiments of the invention, first bio-barrier 152 and second bio-barrier 154 may comprise a multifunctional bio-barrier and include a first impermeable membrane and a second air-permeable membrane.

The first bio-barrier may be designed to have specific microwave and thermal characteristics. For example, the first bio-barrier can be designed to have a loss tangent (tan(δ)) of 0.1 or less, and more particularly, a loss tangent of approximately .0004. In other embodiments, the first bio-barrier may be designed to have an electrical conductivity suitable for use a in a microwave system, such as having a conductivity (σ) of between approximately 0.0 and 0.2 siemens/meter. The first bio-barrier may also be designed to have a thermal conductivity suitable for use in a microwave system, such as having a thermal conductivity of at least approximately 0.1 watts per meter Kelvin (0.1 W/mK), and desirably 0.1 to 0.6 W/mK, and most desirably 0.25 to 0.45 W/mK. Furthermore, the first bio-barrier may be designed to have a heat transfer coefficient suitable for use in a microwave system, such as having a heat transfer coefficient of approximately 7874 W/m2K.

In some embodiments, the first bio-barrier 152 can be designed to conform to a treatment or tissue surface, particularly when a vacuum is applied to the first bio-barrier. In some embodiments, the first bio-barrier can be configured to deflect at least .010 inches with a vacuum of approximately -20 inches of mercury without tearing or deforming. The first bio-barrier may be designed to deflect to cover a treatment or tissue surface without forming bubbles or deformities as such bubbles, voids or deformities may perturb microwave energy passing through first bio-barrier 152, resulting in potential hot spots adjacent the tissue interface surface 200 and/or between bio-barrier 152 and cooling plate 128. In embodiments of the invention a distal surface of tissue cooling plate 128 forms a tissue treatment surface 502 of applicator 114.

When the tissue interface module 116 is placed against tissue, such as the skin, skirt 206 engages the tissue and forms tissue acquisition chamber 142 between the tissue, the skirt, and the first bio-barrier 152. Vacuum can then be applied by the applicator (not shown in this figure) to the tissue interface module to pull tissue into the tissue acquisition chamber 142 and up against the first bio-barrier 152 and tissue interface surface 200. The vacuum can be pulled through vacuum notches 214 surrounding the bio-barrier to achieve vacuum in the tissue acquisition chamber and in the applicator chamber (described below). In the embodiment of Fig. 5, it can be seen that the tissue interface module 116 includes four vacuum notches 214. However, in other embodiments, more or fewer vacuum notches can be implemented around the first bio-barrier. Increasing the number of vacuum notches and positioning the vacuum notches around a perimeter of the bio-barrier can improve vacuum performance in the tissue acquisition chamber and provide vacuum redundancy in the event that one or more of the notches becomes clogged with blood, tissue, or other bodily fluids during treatment.

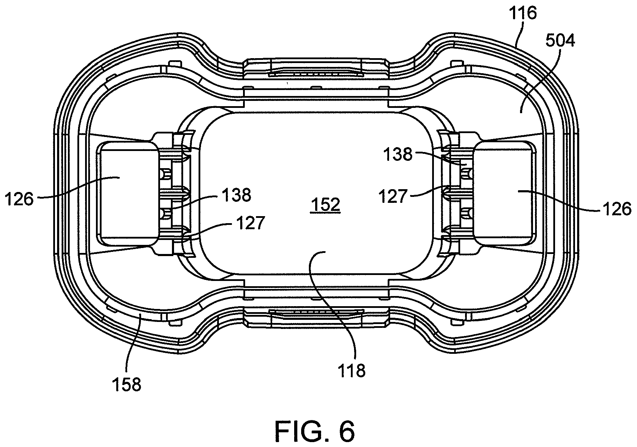

Fig. 6 is a top view of tissue interface module 116 from the non-treatment side of the module which is configured to attach to an applicator, such as, for example, the applicator of Figs. 1-3. In Fig. 6, the tissue interface module 116 includes first bio-barrier 152, applicator chamber 118, attachment mechanism 126 (which may be, for example a ferromagnetic plate), vacuum channels 138, attachment supports 127, and gasket 158. The applicator chamber 118 is adapted to receive and connect to an applicator which includes, for example, a microwave antenna, a cooling element or cooling plate, and at least one vacuum port when the tissue interface module is attached to the applicator (not shown). The gasket can provide a substantially air tight (hermetic) seal against the applicator when the applicator is positioned in the applicator chamber 118. The gasket may have a hardness durometer of, for example, between 20A and 80A. The gasket may also have a thickness of approximately 1/16th of an inch in some embodiments.

The attachment mechanisms can be positioned on the interior or proximal side of the tissue interface module and be adapted to attach the module to the applicator. Connection between the tissue interface module and the applicator may be facilitated by at least one attachment mechanism. In some embodiments, the attachment mechanism may include mechanical elements on the applicator and the tissue interface module. In other embodiments, the attachment mechanisms comprise a metal or ferromagnetic plate configured to attach to a magnet or magnets on the applicator and/or to form a completed magnetic circuit elements of the applicator, including, for example, a positionable magnet and magnet extenders. The attachment mechanism can be medical grade stainless steel plates, for example. In some embodiments, the attachment mechanisms can be magnetic plates having a size of approximately 0.5 inches in width and 1.05 inches in length, with a thickness of approximately 0.63 inches. The size of these plates can, without substantial impact to performance, vary by, for example, plus or minus 20% in other embodiments, for example. Thicker and/or larger magnetic plates can increase the mass of the plate without improving the magnetic holding force, having the undesirable effect of making the tissue interface module more likely to fall or be knocked off the applicator. Thinner and/or smaller magnetic plates can reduce the magnetic holding force, also having the undesirable effect of making the tissue interface module more likely to fall or be knocked off the applicator. The attachment mechanisms can rest upon attachment supports 127, which keep the mechanisms elevated above and prevent the attachment mechanisms from restricting the flow of air through vacuum channels 138 and a second bio-barrier (not shown in this figure). In some embodiments, the attachment supports are adapted to keep the attachment mechanisms raised approximately 0.010 inches above the second bio-barrier(s), optimizing air flow through vacuum channels 138 without substantially increasing the size of the tissue interface module.

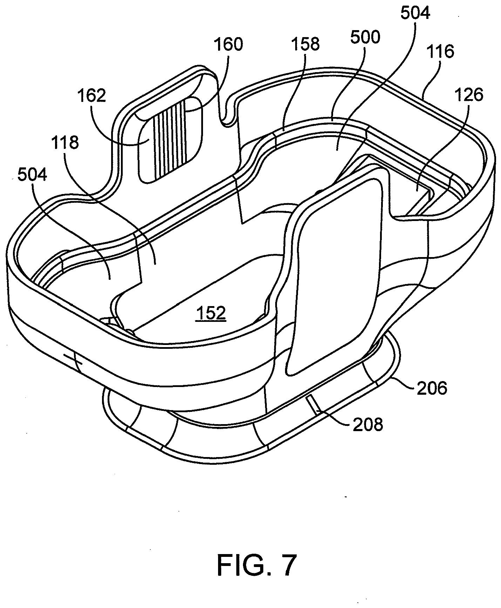

Fig. 7 is a top perspective view of tissue interface module 116, also showing the non-treatment side of the module. Fig. 7 shows the applicator chamber 118, which is adapted to receive and properly position applicator 114 with respect to first bio-barrier 152 when the tissue interface module is attached to the applicator. As described above, the applicator chamber 118 is adapted to receive a microwave antenna, a cooling element or cooling plate, and a vacuum port of the applicator when the tissue interface module is attached to the applicator. Gasket 158 can provide a seal between the module and the applicator when the tissue interface module is attached to the applicator. The opening formed by gasket 158 at the proximal end of applicator chamber 118 may act as a vacuum outlet 504 when tissue interface module 116 is positioned on applicator 114, channeling air to flow out from applicator chamber 118 and into vacuum inlets 174 on applicator 114. An engagement surface 500, which, in one embodiment of the invention may be located at a proximal end of Gasket 158 may be positioned such that engagement surface 500 contacts applicator 114 as tissue interface module 116 is attached to applicator 114. As described above, the interior of the tissue interface module can further include electrodes 160 and printed circuit board 162 configured to, for example, detect proper alignment of the tissue interface module with the applicator.

Also shown are the skirt 206, which is configured to facilitate the engagement of tissue, and alignment marker 208 disposed on the skirt for aligning the tissue interface module with specific portions of the tissue to be treated. During therapy, stamps or markings, including temporary tattoos may be used to mark patient tissue to appropriately place applicators during treatment. Such stamps may be sized to overlay an area to be treated, (e.g. an axilla). A physician may need to select different stamp sizes for different axilla sizes. Stamps are used to mark a number of different treatment points on a patient, including, for example, anesthesia injection sites. Physicians may use the marks created to properly place the applicator before treatment, using, for example the alignment marker 208 on the skirt 206.

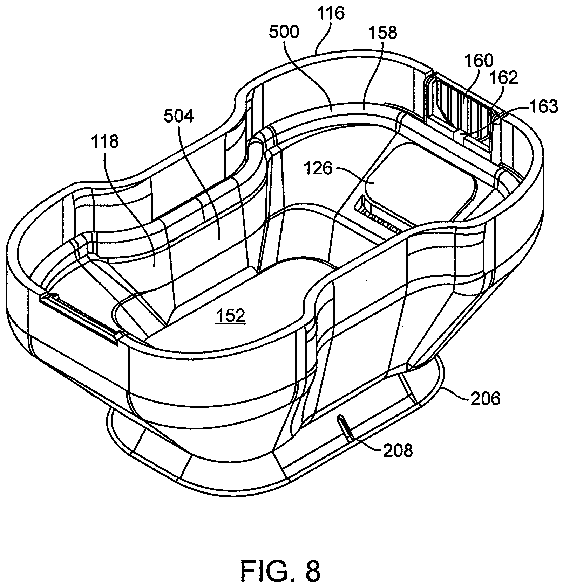

Fig. 8 is a top perspective view of alternate embodiment of a tissue interface module 116. In this embodiment, the printed circuit board 162, electrodes 160, and integrated circuit 163 are positioned on the same side(s) of the module as the attachment mechanism 126. As in Fig. 7, the skirt 206, alignment marker 208, first bio-barrier 152, gasket 158, and applicator chamber 118 can also be seen in this alternative embodiment.

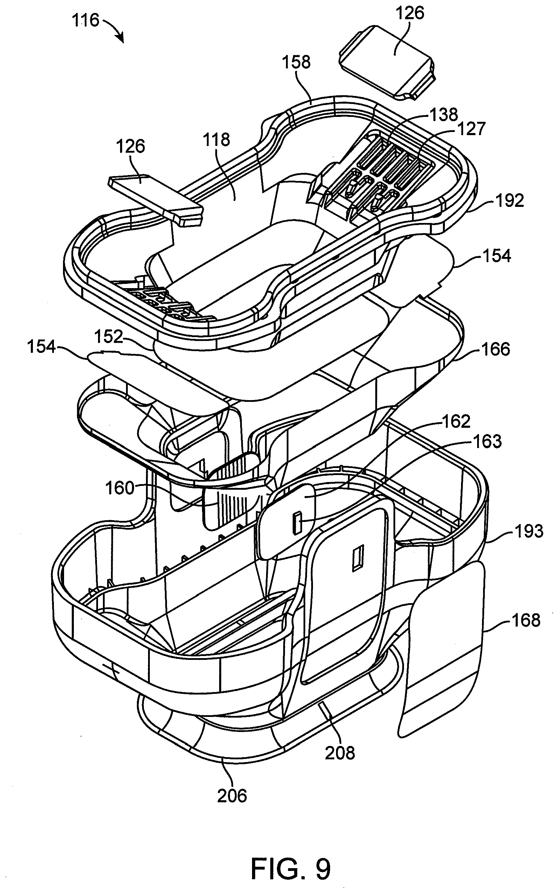

Fig. 9 is an exploded top perspective view of the tissue interface module 116 of Figs. 5-7. In Fig. 9, the tissue interface module 116 can comprise an outer shell 193 and an inner insert 192. The inner insert can comprise first bio-barrier 152, second bio-barriers 154, attachment mechanisms 126, gasket 158, vacuum channels 138, attachment supports 127 and applicator chamber 118. Shell 193 can include electrodes 160, printed circuit board 162, integrated circuit 163, cover 168, alignment marker 208 and skirt 206.

As shown in Fig. 9, one or more second bio-barriers 154 can be positioned on both sides of the first bio-barrier 152. Membranes or filters suitable for use as second bio-barriers 154 may include membranes which are permeable to air but substantially impermeable to biological fluids. As will be described in more detail below, when vacuum is pulled from the applicator through the applicator chamber, the second bio-barriers 154 allow air or gas but not fluid or tissue to pass, thereby allowing vacuum to be created in the tissue acquisition chamber, which pulls air from the tissue chamber through the second bio-barriers to engage the first bio-barrier 152 and the tissue interface surface with the tissue to be treated.

In some embodiments, the second bio-barriers made from hydrophobic material. In other embodiments, the second bio-barriers have a pore size to area ratio of a predetermined value to allow for passage of gas or air but not of liquids such as blood and sweat. In some embodiments, the second bio-barriers may have a size and pore size such that the overall opening facilitates the equalization of pressure across such second bio-barrier within approximately one half second (to a maximum of three seconds) as tissue is drawn into the tissue acquisition chamber 142. In another embodiment, the second bio-barriers may be positioned such that a vacuum in the tissue acquisition chamber is less than a vacuum in the applicator chamber as air is drawn from the tissue chamber and the applicator chamber, facilitating the positioning of a first bio-barrier against the treatment surface of the applicator. In other embodiments, the second bio-barriers may have a flow rate of a predetermined value when vacuum is applied so as to help equalize pressure across the bio-barriers. In one embodiment, the second bio-barriers can have pore sizes of approximately .45 um and a flow area of a predetermined number of square inches. The second bio-barrier may be, for example, PTFE on a polyester backing, polyethylene film, nylon or other material meeting the criteria set forth above.

Referring still to Fig. 9, reflector 166 can optionally be positioned between the inner insert and the outer shell, or integrated into the inner insert, the outer shell, or both. The reflector can comprise an electrically conductive mesh with predetermined openings so as to improve performance of energy delivery from the applicator to tissue by reflecting microwave energy. In some embodiments, the reflector is configured to isolate stray electromagnetic fields and reflect stray electromagnetic energy back into the applicator. In some embodiments, the reflector is positioned so as to be electrically isolated from an applicator and electrically isolated from tissue positioned in the tissue acquisition chamber. The reflector can be sized and configured to substantially surround a tissue interface surface of the module. In some embodiments, the reflector can comprise a metallic mesh material of wire having a diameter of approximately 0.008 inches with wires arranged to be approximately 30 by 30 wires per inch.

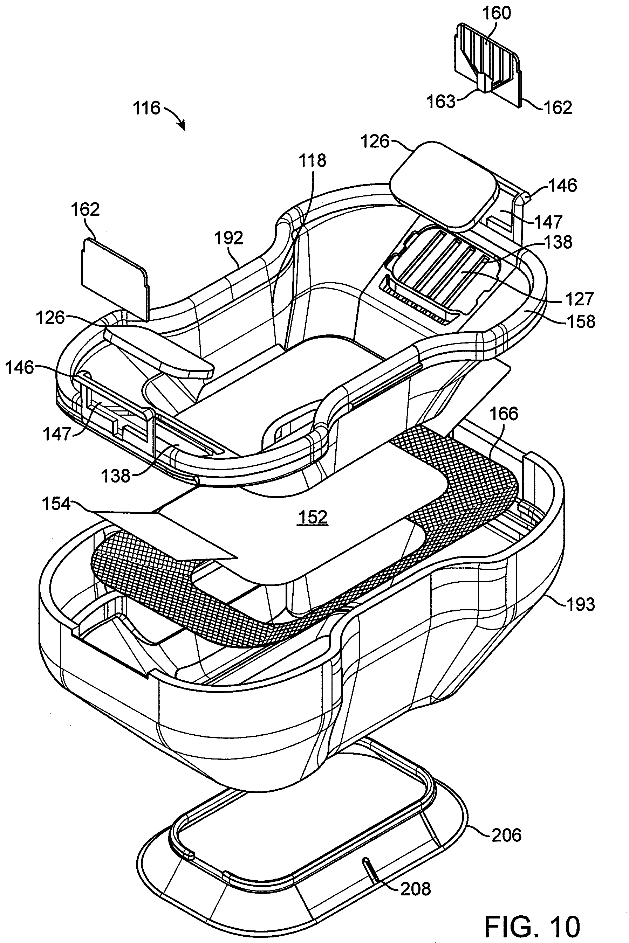

Fig. 10 is an exploded top perspective view of the tissue interface module 116 of Fig. 8. In Fig. 10, the tissue interface module 116 can comprise an outer shell 193 and an inner insert 192. The inner insert can comprise first bio-barrier 152, second bio-barrier(s) 154, attachment mechanisms 126, gasket 158, vacuum channels 138, attachment supports 127, applicator chamber 118, electrodes 160, printed circuit board 162, integrated circuit 163, tab member 146, and latch openings 147. Shell 193 can include alignment marker 208 and skirt 206. Reflector 166 can optionally be positioned between the inner insert and the outer shell, or integrated into the inner insert, the outer shell, or both.

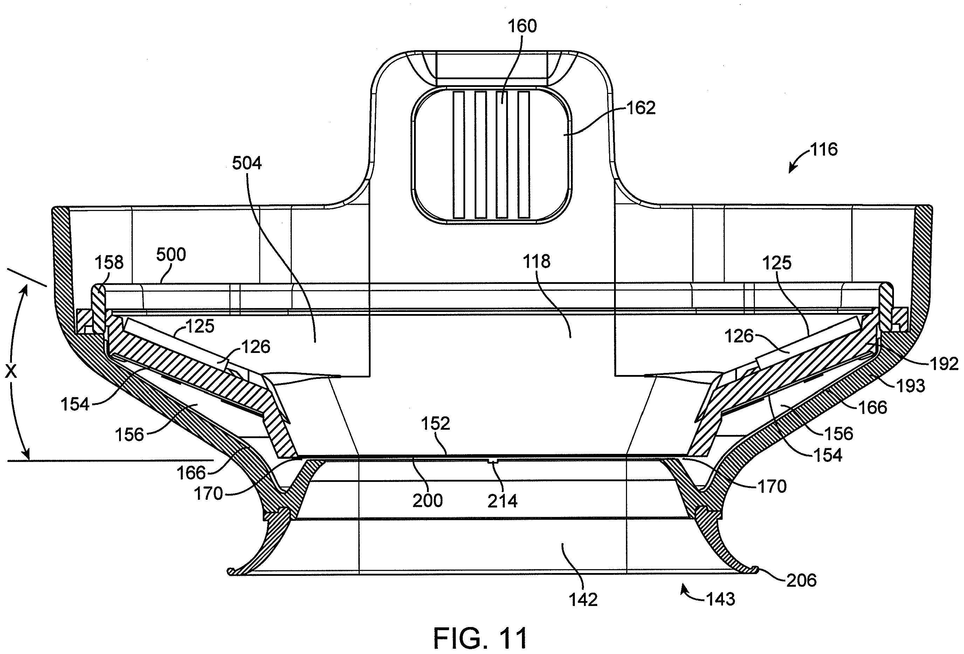

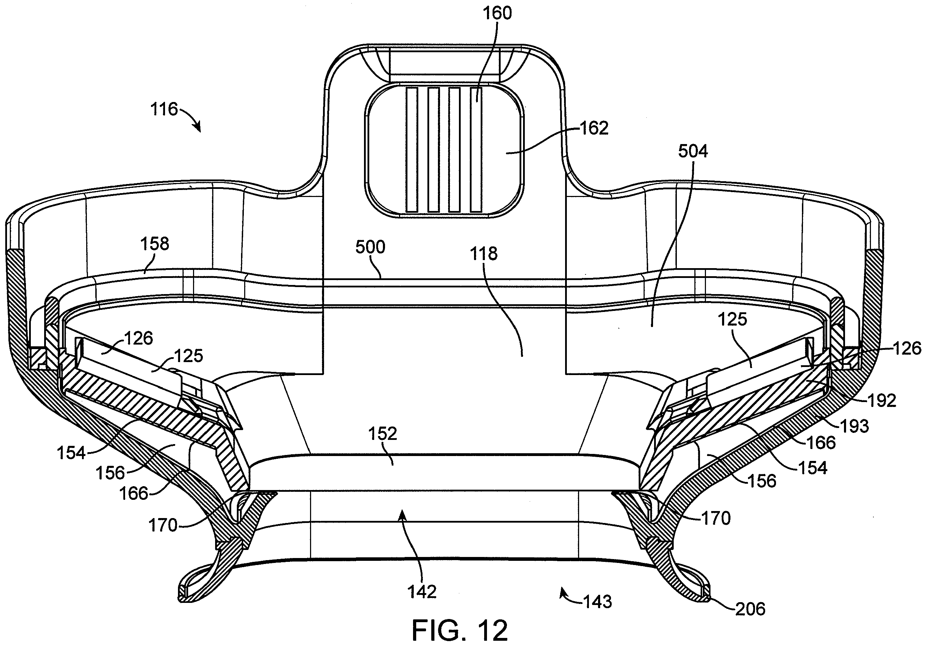

Fig. 11 illustrates a side cutaway view of a tissue interface module 116, and Fig. 12 shows a side cutaway perspective view of the tissue interface module. The module of Figs. 11-12 can include many of the features described above, including tissue acquisition chamber 142, first bio-barrier 152, second bio-barriers 154, applicator chamber 118, electrodes 160, printed circuit board 162, attachment mechanism 126, gasket 158, inner insert 192, outer shell 193, reflector 166, skirt 206, acquisition chamber opening 143, vacuum notches 214, tissue interface surface 200, and consumable gasket 158. Attachment mechanisms 126 include engagement surface 125, which is configured to attach to the applicator (e.g., via magnetic attachment). In some embodiments, engagement surface 125 forms an angle of approximately 22.5 degrees from horizontal (e.g., from a plane through first bio-barrier 152) so as to couple to an applicator having attachment points comprising the same angle. In other embodiments, engagement surface 125 forms an angle of approximately 17.5 degrees to 27.5 degrees from horizontal (e.g., from first bio-barrier 152), or alternatively, from approximately 12.5 degrees to 32.5 degrees. In other embodiments, the angle of attachment can vary, up to and including 45 degrees or more.

As described above, the attachment mechanisms can be disposed on attachment supports over vacuum channels (not shown in Fig. 11). The second bio-barriers 154 can be positioned on the other side of the attachment supports and vacuum channels. In addition to the features described above, the tissue interface module 116 can further include fluid traps 156 integrated into the module and an expandable aperture (also referred to as a variable flow restrictor) 170 between the tissue acquisition chamber 142 and the fluid traps 156.

The fluid traps 156 are configured to collect blood, sweat, and any other bodily fluids or tissue that may collect within the tissue interface module during treatment. By collecting bodily fluids or tissues in the fluid traps 156, the tissue interface module is able to keep the second bio-barriers clear from obstructions that would otherwise interfere with treatment or render treatment impossible. Thus, the second bio-barriers are disposed between, and communicating with, both the applicator chamber 118 and the tissue acquisition chamber 142. As described above, the second bio-barriers 154 can comprise openings configured to permit air or gas to pass but prevent liquid from passing through the second bio-barriers. The applicator chamber 118 is able to communicate with the tissue acquisition chamber 142 via second bio-barriers 154 and vacuum channels (vacuum channels 138 of Figs. 6 and 9).

The expandable aperture 170 may be included at a proximal end of the tissue acquisition chamber, and the aperture may comprise, for example, a gap at top of the tissue acquisition chamber between a first bio-barrier and an interior rim of the tissue acquisition chamber. Notches may be included in the tissue acquisition chamber proximal to the gap to enhance vacuum acquisition. In some embodiments, one wall of the aperture may be flexible to increase in size and airflow when vacuum is applied. A tissue interface surface 200 of the applicator may act to restrict the width of the aperture as it expands. A suitable aperture is sized to allow air to pass into a vacuum path while preventing tissue from blocking such vacuum path.

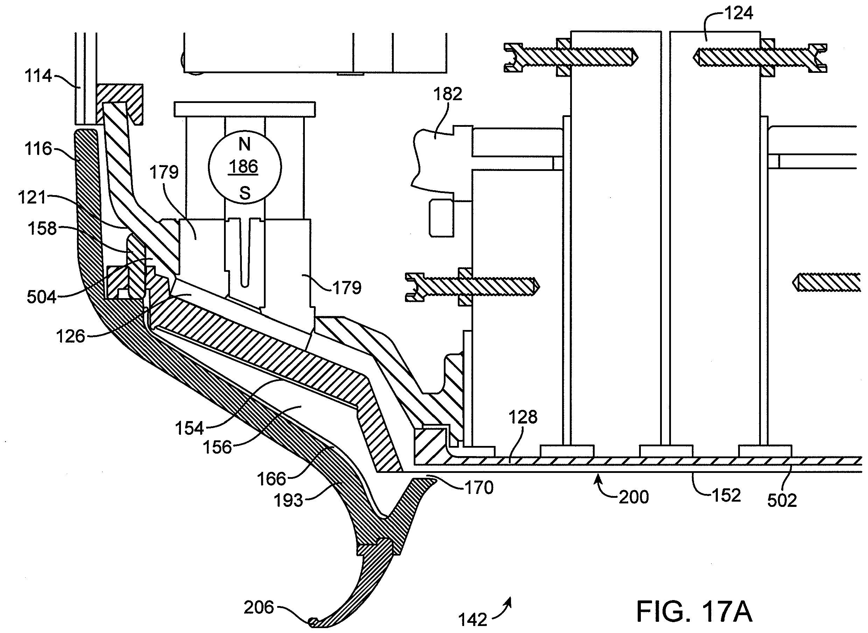

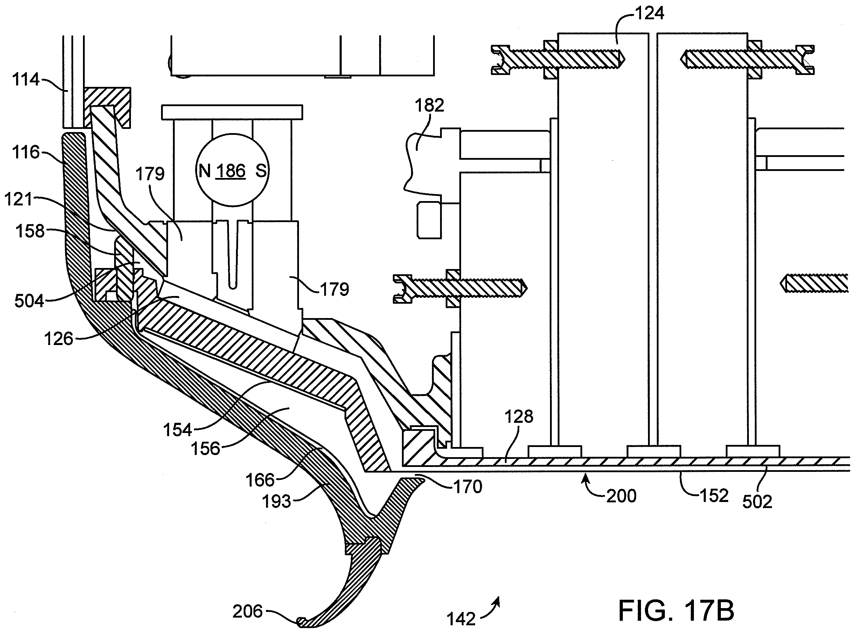

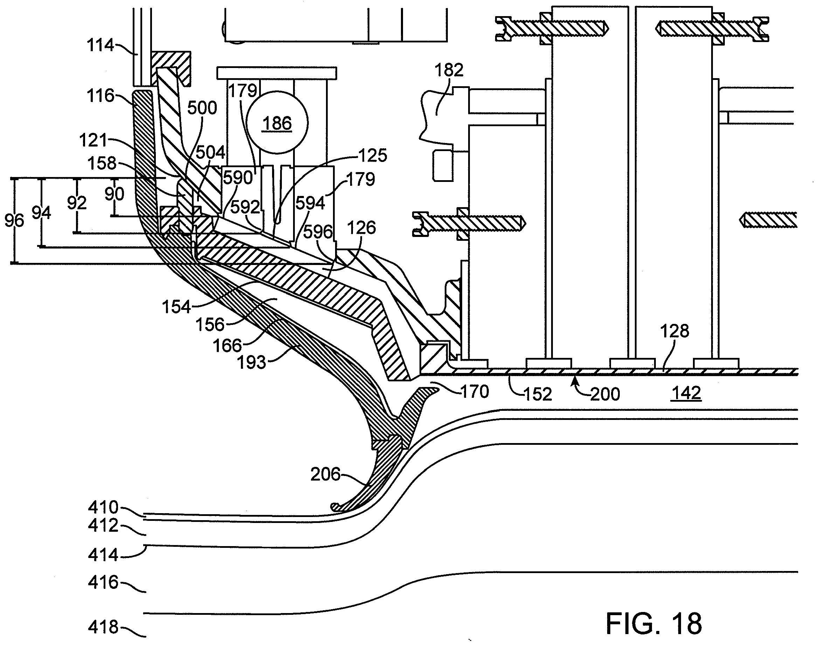

Expandable aperture 170 can be configured to expand when vacuum is applied by an applicator (not shown) to the applicator chamber 118 and to the tissue acquisition chamber 142. The application of vacuum to the tissue interface module can pull the first bio-barrier 152 inwards towards the cooling plate of the applicator, which increases the size of expandable aperture 170. Figures 17A and 17B illustrate embodiments of the invention wherein first bio-barrier 152 is in its inflexed state and expandable aperture 170 is at its minimum width. In Figure 18, expandable aperture 170 has been opened to its maximum width by the application of vacuum pressure to applicator chamber 118, which pulls bio-barrier 152 against tissue treatment surface 502, which, in one embodiment may be cooling plate 128, opening aperture 170. As tissue is pulled into and air is pulled out of tissue chamber 142 a small vacuum pressure differential is maintained by the drop in pressure across second bio-barrier 154 such that the pressure in applicator chamber 118 is less than the pressure in tissue chamber 142. This pressure differential may be used to, for example, maintain the position of bio-barrier 152 against cooling plate 128 during the acquisition of tissue. This pressure differential may further be used to ensure that bio-barrier 152 is positioned against cooling plate 128 prior to tissue contacting tissue interface surface 200 which may, for example, ensure that bio-barrier 152 is positioned against cooling plate 128 without bubbles, voids or deformities and/or that tissue being pulled into tissue chamber 142 does not move or deform bio-barrier 152. Once the air is removed from tissue chamber 142 and replaced by tissue, air will no longer flow into applicator chamber 118 and the pressure in the two chambers will be substantially balanced. With tissue properly positioned in applicator chamber 118, the tissue pressing against tissue interface surface 200 will act to maintain the position of bio-barrier 152 against cooling plate 128, preventing the formation of voids, bubbles or deformities which could result in hot spots.

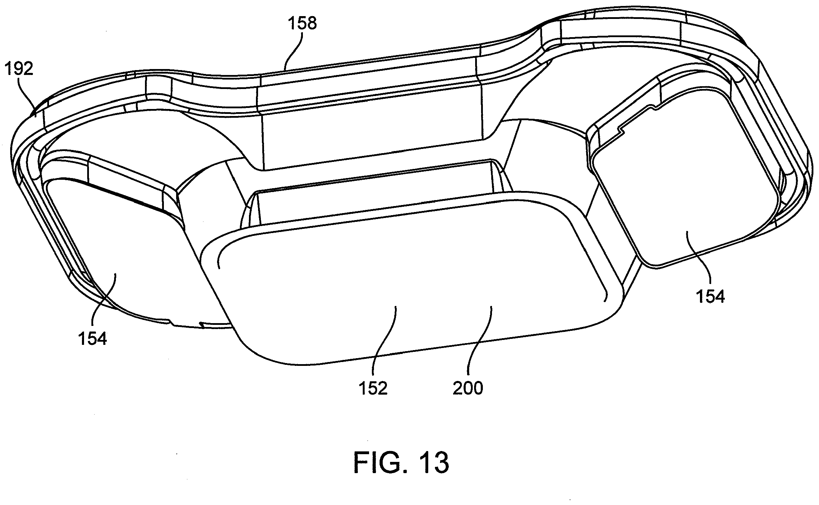

Fig. 13 illustrates a perspective end view of inner insert 192, showing the first bio-barrier 152, second bio-barriers 154, and gasket 158. This view of the inner insert shows the portions of second bio-barriers 154 which interface with and help form the fluid traps described in Figs. 11-12. As shown in Fig. 13, the second bio-barriers can be sized to occupy most of the remaining space on the inner insert besides the first bio-barrier 154. Maximizing the surface area of the second bio-barriers can increase vacuum performance and provide redundancy in case one of the second bio-barriers becomes clogged with tissue or bodily fluids. In the illustrated embodiment, the second bio-barriers can occupy approximately 50% of the surface area of the inner insert 192, with the first bio-barrier occupying approximately the remaining surface area. In other embodiments, the first bio-barrier can occupy approximately 50-70% of the surface area of the inner insert, and the second bio-barriers can occupy the remaining 30-50% of the surface area of the inner insert.

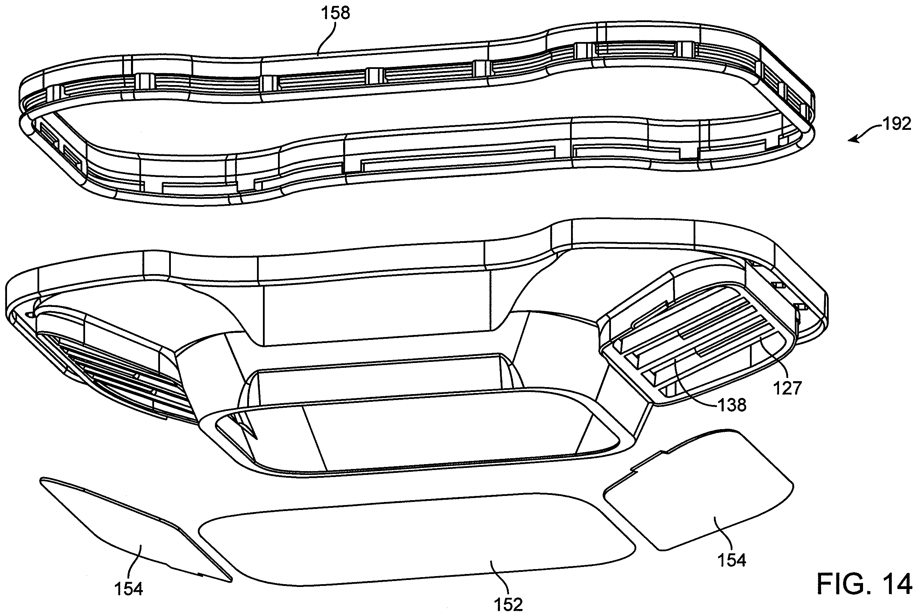

Fig. 14 is an exploded perspective side view of inner insert 192, revealing vacuum channels 138 and attachment supports 127 behind the second-bio barriers 154. As described above, the vacuum channels 138 allow for airflow under the attachment mechanisms (not shown) and through the second bio-barriers, to allow for vacuum communication between the applicator chamber and the tissue acquisition chamber of the tissue interface module.

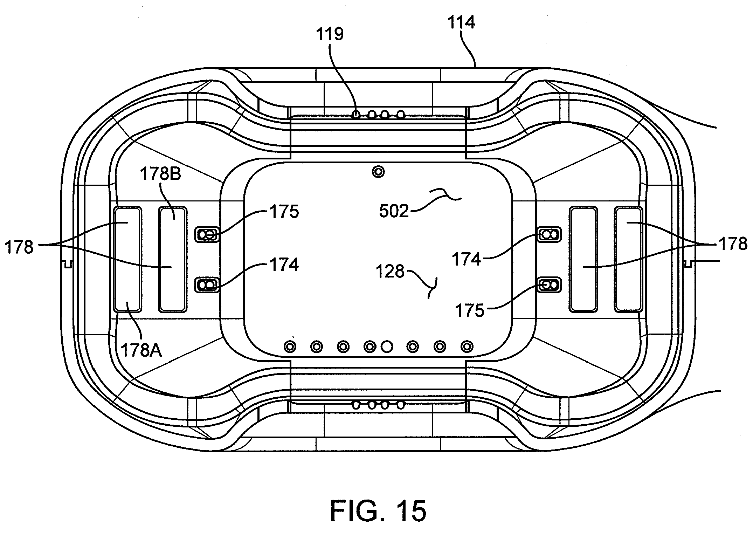

Fig. 15 shows an end view of applicator 114 without tissue interface module 116 attached. The applicator 114 can include electrical contacts 119 for electrical coupling with the electrodes and printed circuit board of the module, cooling plate 128, applicator vacuum inlets 174, aesthetic features 175 and engagement surface 178 configured to engage the attachment mechanisms of the tissue interface module. The applicator vacuum inlets 174 are coupled to a vacuum source (not shown). When the tissue interface module is attached to the applicator of Fig. 15, the applicator vacuum inlets are configured be received by the applicator chamber and to pull a vacuum through the applicator chamber, through the second bio-barriers, and then through the tissue acquisition chamber, as described above.

The cooling plate 128 of the applicator may include an alumina or other metal frame surrounding the back side of the plate to add structural strength to the plate, a plurality (e.g. four) of threaded rods may be bonded to the alumina frame to the plate to a waveguide holder (not shown). In some embodiments, the plate can comprise a ceramic material having approximately 96% alumina and 4% other material. The cooling plate may further include one or more thermocouple traces of, for example, copper and constantan for detecting a temperature of the cooling plate or of the tissue to be treated. Such traces may be routed in side by side pairs to, for example, reduce the effect of noise on the output of such thermocouples. When the applicator is attached to the tissue interface module, applying vacuum to the module can result in pulling the first bio-barrier of the module against the cooling plate of the applicator.

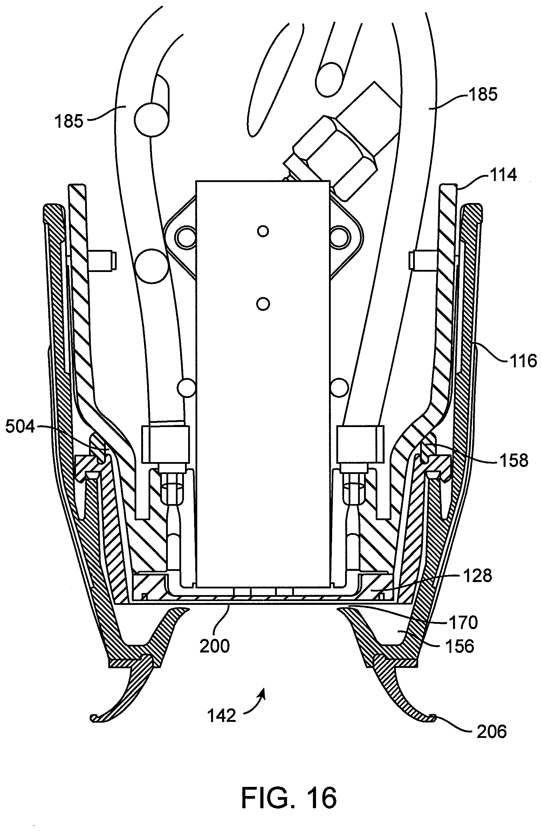

Fig. 16 shows a side cutaway view of a section of applicator 114 and a portion of tissue interface module 116 attached to the applicator. In Fig. 16, the side angle shows how skirt 206 forms the tissue acquisition chamber 142 and tissue interface surface 200. Fig. 16 also reveals the vacuum flow path from tissue acquisition chamber 142 through expandable aperture 170 into fluid trap 156. Also shown are coolant conduits 185 of applicator 114, which supply cooling fluid to the applicator cooling plate described above. The coolant conduits can comprise antimicrobial fittings and tubing using AglOntm technologies. Such fittings and tubing may provide protection against microbial colonization (e.g. bacteria, mildew, mold and fungi. The tubing may also be adapted to provide protection against microbial colonization without impacting, reducing or modifying the microwave characteristics (e.g. loss characteristics) of cooling fluid passing through such antimicrobial fittings and tubing.

Figs. 17A-17B are zoomed-in side cutaway views of applicator 114 and tissue interface module 116 attached to the applicator. In Figs. 17A-17B, applicator 114 includes rotatable magnet 186 which is configured to complete a magnetic circuit between magnetic extenders 179 and attachment mechanism 126. Completing the magnetic circuit between the magnetic extenders and the attachment mechanism magnetically couples attachment mechanism 126 on the tissue interface module to magnetic extenders 179 on the applicator. Magnet 186 can be coupled to a rotation mechanism such as a direct current gear motor or an RC servomotor, so as to rotate the magnet within magnetic extenders 179 between an incomplete magnetic circuit and a completed magnetic circuit. In Fig. 17A, the "N" and "S" poles of magnet 186 are shown in the vertical position, and therefore do not complete a magnetic circuit with magnetic extenders 179 and attachment mechanism 126. When the magnetic circuit is incomplete, there is little or no magnetic attraction between the attachment mechanism and the magnetic extenders, allowing for removal of the tissue interface module from the applicator. In Fig. 17B, the "N" and "S" poles of magnet 186 have been rotated into the horizontal position, thereby completing the magnetic circuit and magnetically attaching the magnetic extenders to the attachment mechanism. In some embodiments, a stop may be implemented using a hall effect position sensor or a hard stop.

Other features of the tissue interface module already described above but shown in Figs. 17A-17B include gasket 158, expandable aperture 170, integrated fluid trap 156, skirt 206, tissue acquisition chamber 142, first bio-barrier 152, second bio-barrier 154, outer shell 193, reflector 166, and attachment mechanisms 126. Also shown, a gasket contact surface of the applicator 114 may be angled to receive the gasket 158 from the tissue interface module. In this embodiment, since the seal is placed at an angle, the gasket bends when the tissue interface module attaches to the applicator, improving the sealing characteristics and reducing the force required to attach the tissue interface module to the applicator.

Fig. 18 illustrates the applicator and tissue interface module of Figs. 17A-17B placed in contact with tissue with treatment and vacuum initiated. In Fig. 18, the tissue, including epidermis 410, dermis 412, dermal-hypodermal interface 414, hypodermis 416, and muscle 418 are shown being pulled by vacuum into tissue acquisition chamber 142 towards the tissue interface surface 200 and bio-barrier 152. Vacuum pressure applied from the applicator to the applicator chamber of the tissue interface module can be adapted to localize and stabilize tissue located in the tissue acquisition chamber 142. The vacuum pressure is also adapted to hold tissue positioned in the tissue acquisition chamber against the tissue interface surface 200 and the first bio-barrier 152 of applicator 114. Additionally, vacuum in the applicator chamber of pulls the bio-barrier 152 into contact with cooling plate 128, so as to cool the target tissue during application of microwave energy. In some embodiments, the vacuum is configured to have a flow rate of approximately 13.7 Standard Fluid Liters Per Minute during treatment.

In Fig. 18, applicator 114 includes magnetic extenders 179 and cooling plate 128. Consumable 116 includes consumable gasket 158, expandable aperture 170, integrated fluid trap 156, skirt 206, tissue acquisition chamber 142, first bio-barrier 152, second bio-barrier 154, shell 193, reflector 166, consumable latch plate 126 and tissue interface surface 200. Tissue, including epidermis 410, dermis 412, dermal-hypodermal interface 414, hypodermis 416 and muscle 418 are shown positioned partially within tissue acquisition chamber 142.

In one particular embodiment, as shown in Fig. 18: the vertical distance 90 from engagement surface 500 (which in this embodiment may be the top of gasket 158) to a connection point 590 on engagement surface 125 of the uppermost portion of attachment mechanism 126 is approximately 0.15". In one embodiment, the vertical distance 92 from engagement surface 500 to a connection point 592 on engagement surface 125 of the portion of attachment mechanism 126 that intersects the inside of the left magnetic extender is approximately 0.22". In one embodiment the vertical distance 94 from engagement surface 500 to a connection point 594 on engagement surface 125 of the portion of attachment mechanism 126 that intersects the inside of the right magnetic extender is approximately 0.27". And, in a further embodiment, the vertical distance 96 from engagement surface 500 to a connection point 596 on at the lower portion of engagement surface 125 of attachment mechanism 126 is approximately 0.34". In some embodiments these measurements can vary by ± 0.01". In some embodiments, these measurements can vary by ± 0.05". In one embodiment, the angle of attachment mechanism 126 can be identical to the angle of magnetic extenders 179 to provide a flush fit between the extenders and the attachment mechanism when the tissue interface module is attached to the applicator.

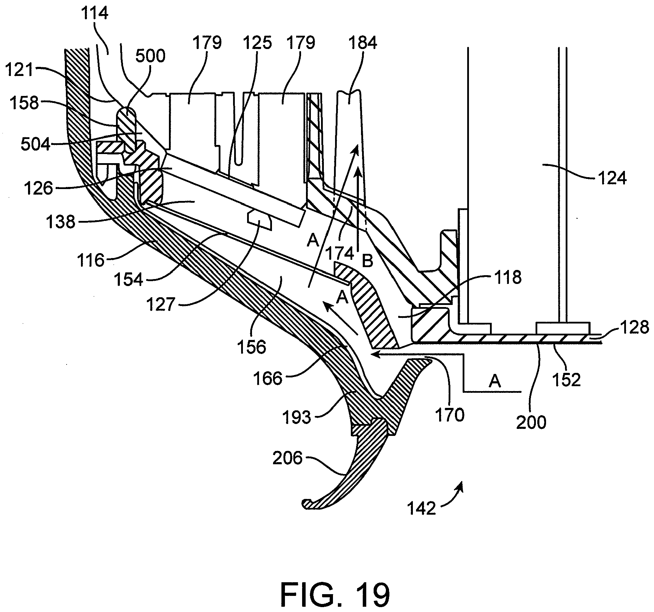

Fig. 19 is a side cutaway view of the applicator and tissue interface module of Figs. 17-18 showing air paths A and B through the tissue interface module with vacuum applied. As shown, vacuum can be applied directly by the applicator 114 to applicator chamber 118 of the tissue interface module 116 to create vacuum within the applicator chamber, as well as within the tissue interface chamber 142. A first vacuum flow path A flows from the tissue interface chamber 142, through expandable aperture 170 into the fluid trap156, and through second bio-barrier(s) 154 into the applicator chamber 118 and into the applicator itself. Second vacuum flow path B shows vacuum being pulled directly from the applicator chamber 118 into the applicator. When vacuum is created along flow path A, tissue is pulled into the tissue acquisition chamber 142, as shown above in Fig. 18. Any tissue or bodily fluids, such as blood or sweat, collect in fluid trap 156 but are unable to pass through second bio-barrier 154. Since the second bio-barriers 154 are permeable to air or gas but not to liquid, vacuum can pulled through the second bio-barriers despite the collection of tissue or bodily fluids in the fluid traps. The vacuum air paths A and B can be used to equalize or substantially equalize pressure on both sides of the tissue interface surface 200 (i.e., in the tissue acquisition chamber 142 and the applicator chamber 118).

In some embodiments, the vacuum flow path is completely internal to the tissue interface module 116 and applicator 114, originating in the applicator itself, and pulling vacuum from the applicator chamber 118, through the second bio-barriers 154, through the fluid traps 156, through the expandable aperture 170, and finally through the tissue interface chamber 142 to engage tissue. In many embodiments, the vacuum flow path hooks up directly from the applicator chamber of the tissue interface module to the vacuum ports of the applicator, without requiring an external attachment from the tissue interface module to the applicator or to a vacuum source (e.g., a tube connecting vacuum to the tissue interface module). In one embodiment, the vacuum path may include at least one portion thereof having a gap width of approximately 0.020 inches.

Vacuum may be achieved and maintained when the tissue interface module 116 is attached to the applicator 114 and tissue is engaged by the tissue acquisition chamber (as shown in Fig. 18). A consumable may have one or more vacuum balance pathways designed therein. One vacuum balance path might consist of a tissue acquisition chamber, a vacuum reservoir and at least one bio-barrier adapted to allow air to pass without allowing other fluids to pass. A vacuum reservoir entrance may also be included and may be flexible to allow the entrance to open, creating a wider gap when vacuum is applied. A reflector may further be included in the vacuum path as, for example, a portion of a vacuum reservoir.

Vacuum in the system 110 may be balanced by including a second vacuum path which runs to an applicator chamber. An applicator chamber may be designed and configured to allow the applicator, when inserted into the applicator chamber to form an airtight seal around the applicator chamber (e.g. with a gasket positioned in the consumable) and to position a distal end of the applicator (e.g. the cooling plate application surface) within .010 inches of the bio-barrier. First and second balance paths may combine in the applicator chamber. Air being evacuated from the tissue chamber, through the second bio-barrier may flow past one or more magnetic plates.

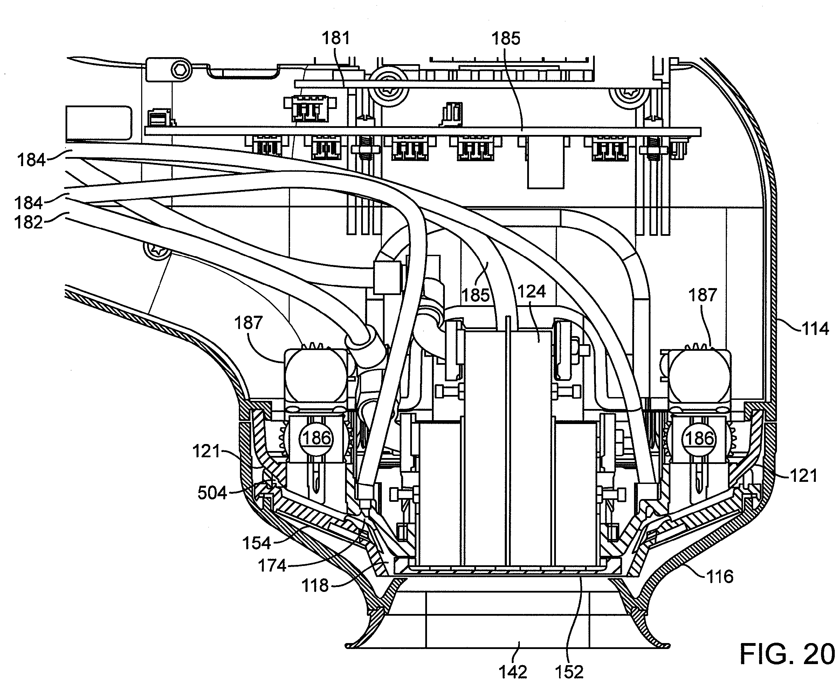

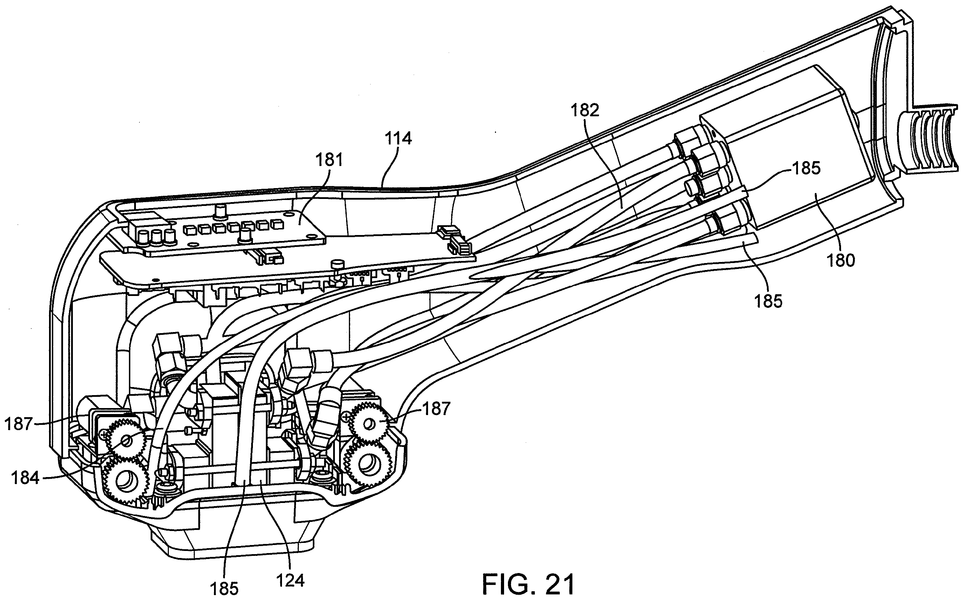

Figs. 20-21 show a side cutaway view and a side cutaway perspective view, respectively, of the internal components of applicator 114, including applicator logic circuits 181, microwave feed cables 182, coolant conduits 185, vacuum conduits 184, antenna array 124, microwave switch 180 and magnetic drive 186. As shown, the applicator chamber 118 of the tissue interface module is adapted and configured to receive the microwave feed cables 182, the cooling conduits 185, and vacuum conduits 184 of the applicator.

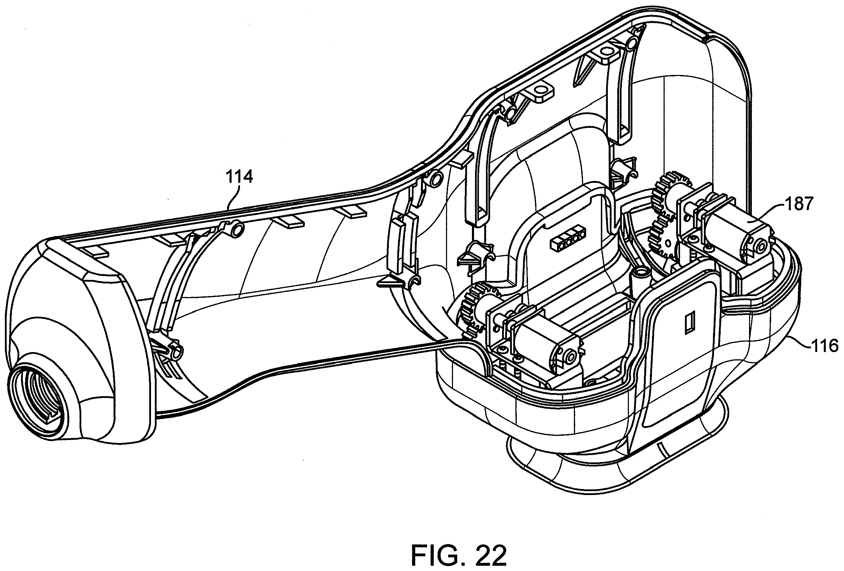

Fig. 22 is a side cutaway perspective view of applicator 114 with consumable 116 attached showing a portion of magnetic drive components, including magnetic drive 186. As described above in Figs. 17A-17B, the magnetic drive 186 can complete a magnetic circuit within magnetic extenders to magnetically attach the tissue interface module to the applicator.

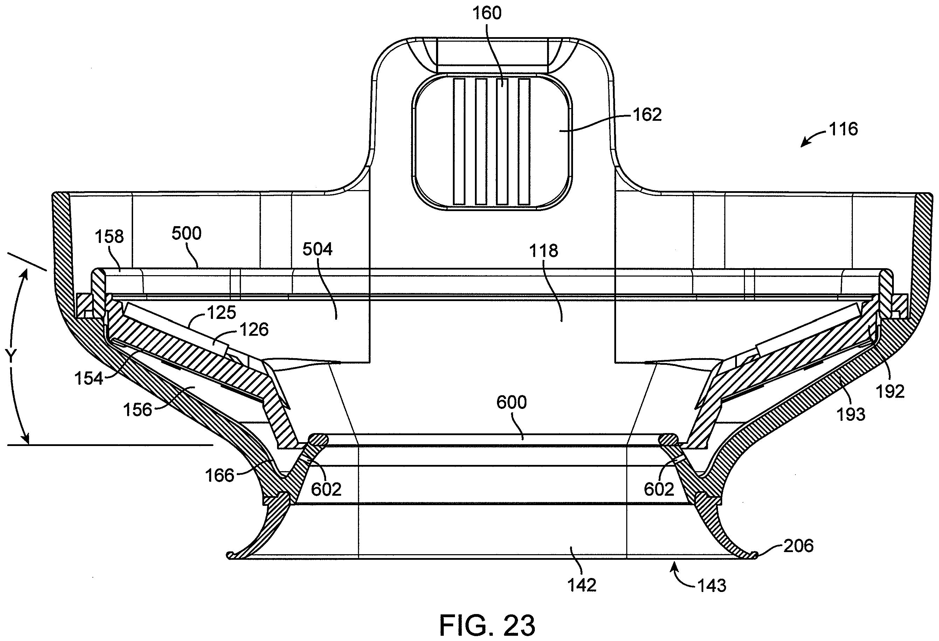

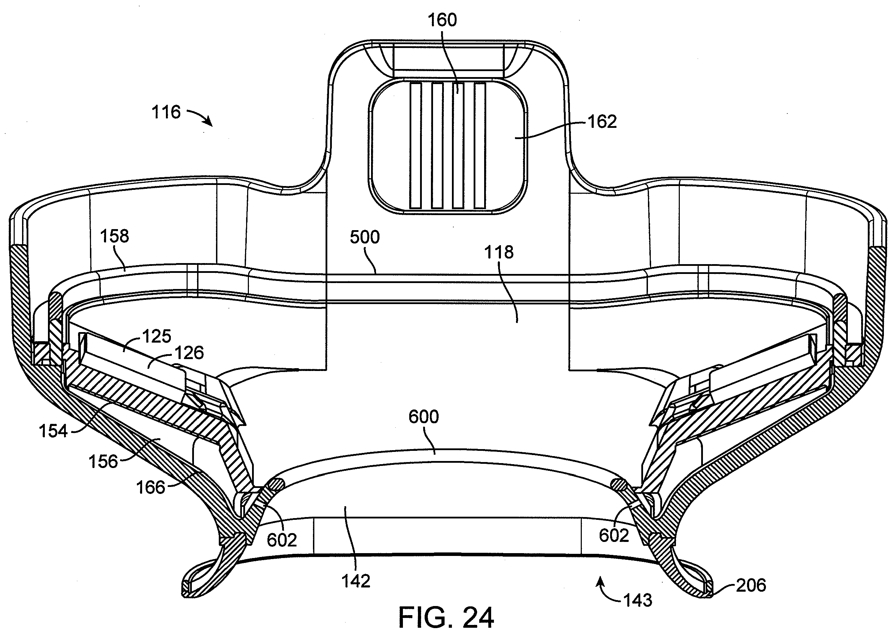

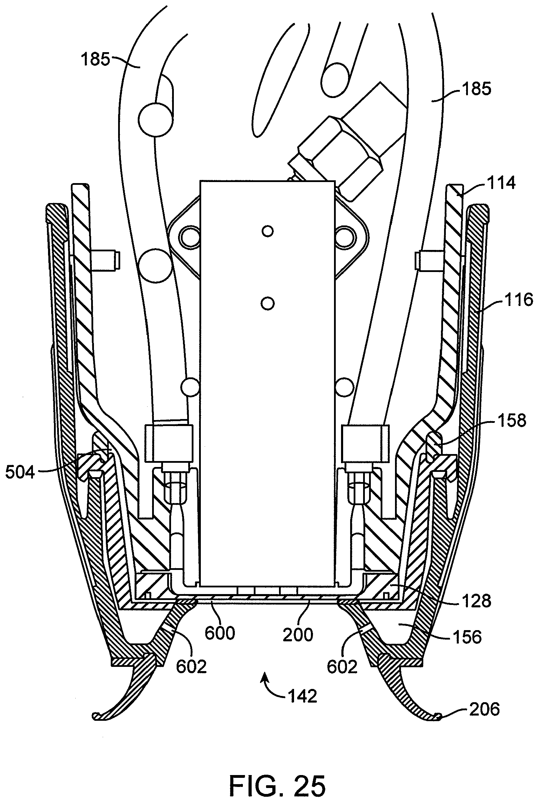

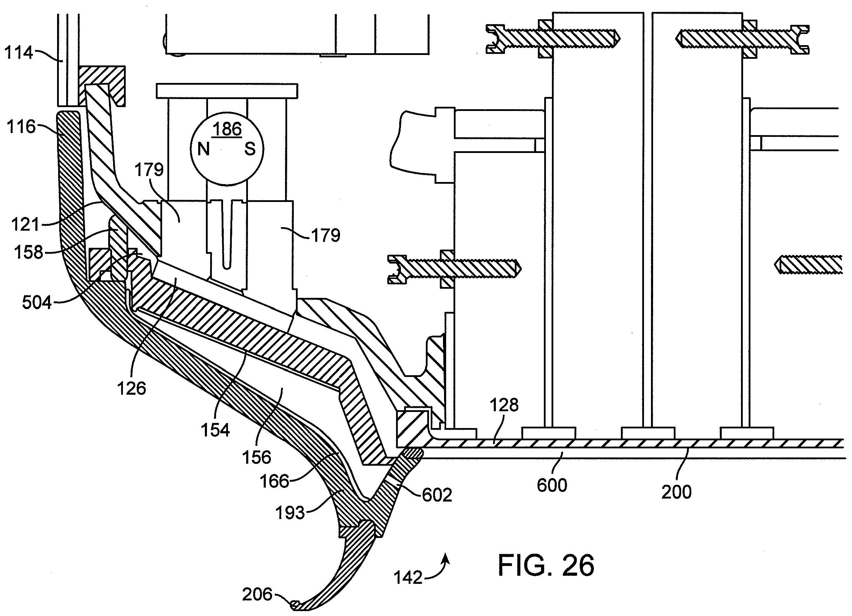

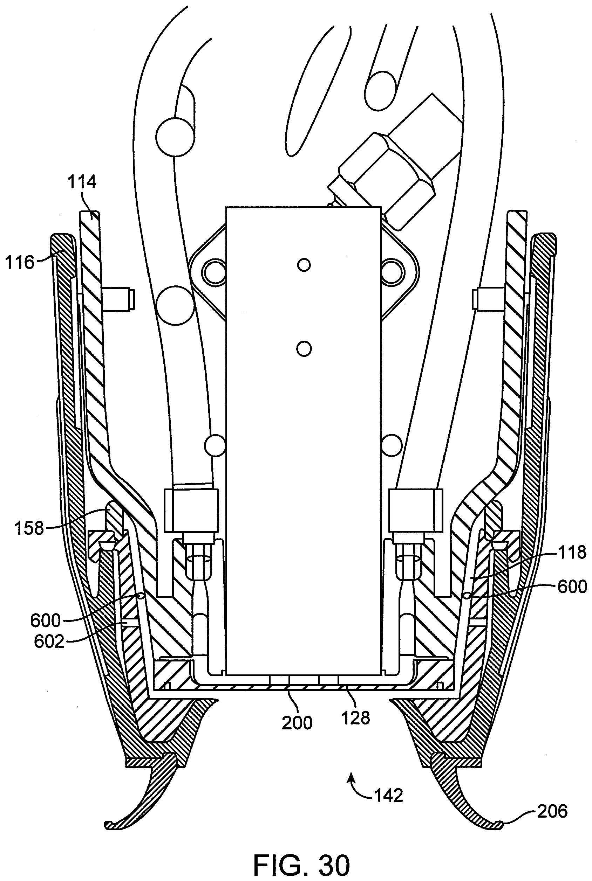

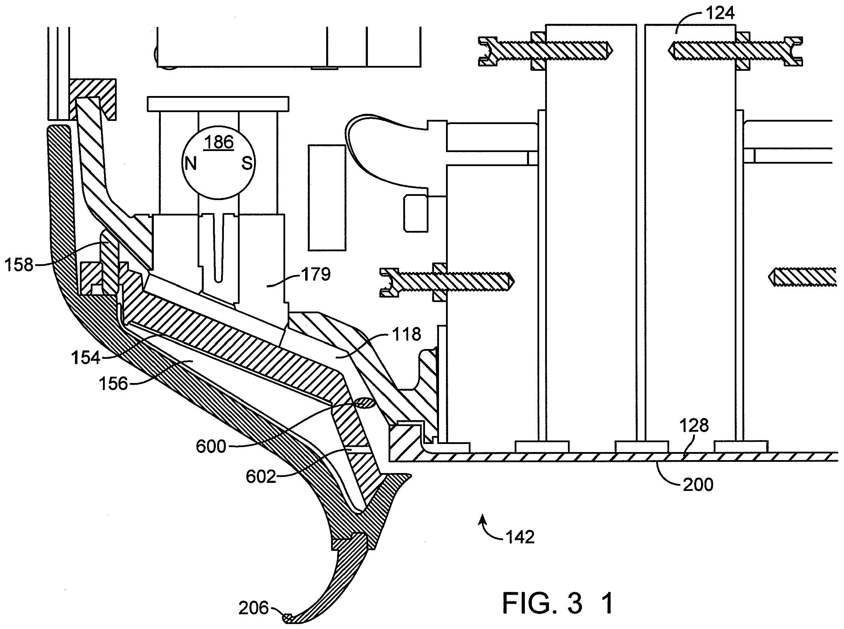

The embodiments of the tissue interface modules illustrated in Figs. 23 to 32 may include many of the features described herein with respect to prior described embodiments, including tissue interface module 116, applicator 114, vacuum channels 138, tissue acquisition chamber 142, filters 154, applicator chamber 118, electrical contacts 160, printed circuit board 162, attachment mechanism 126, engagement surfaces 125, gasket 158, gasket engagement surface 500, inner insert 192, outer shell 193, reflector 166, fluid trap 156, skirt 206, acquisition chamber opening 143 and vacuum interface 504 but omit the flexible bio-barrier 152 shown in earlier embodiments. Embodiments of tissue interface modules illustrated in Figs. 23 through 32 may further include an intermediate gasket 600 (which may also be referred to as an intermediate sealing member) and one or more air passages 602 extending between tissue acquisition chamber 142 and fluid trap 156. Embodiments of applicators illustrated in Figs. 23 to 32 may include many of the features described herein with respect to prior described embodiments, including cooling plate 128, applicator vacuum inlets 174, coolant conduits 185, tissue interface surface 200, magnetic extenders 179, magnet 186, sealing surface 121 and vacuum conduits 184. Tissue interface surface 200 may, in some embodiments of the invention comprise at least a portion of applicator tissue treatment surface 502.

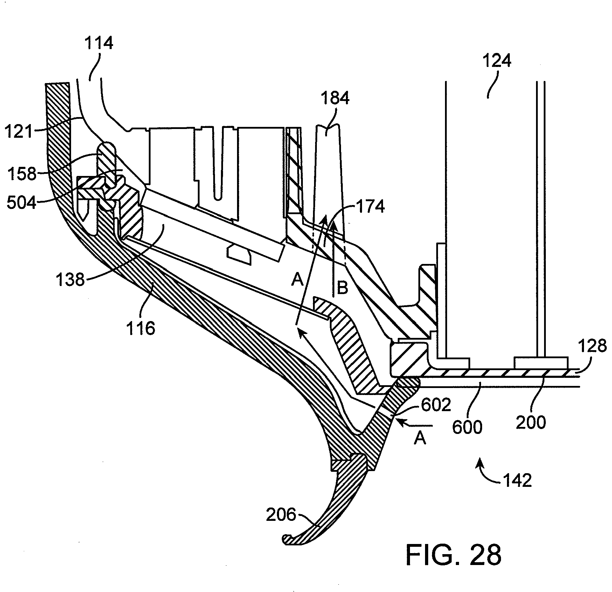

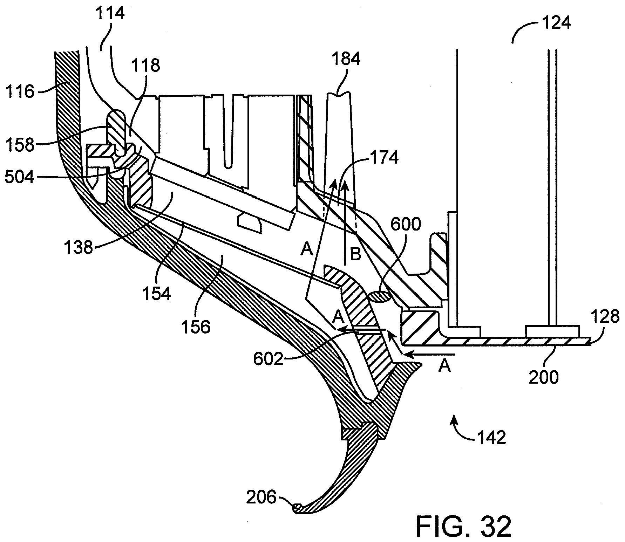

In embodiments of the invention, such as, for example, the embodiments illustrated in Figs. 23 to 32, when tissue interface module 116 is attached to applicator 114, applicator vacuum inlets 174 are configured to be positioned in applicator chamber 118 (see, e.g., Figs. 28 and 32) and to evacuate air from applicator chamber 118 through, for example, vacuum interface 504, creating a vacuum in applicator chamber 118. When positioned on applicator 114, embodiments of the tissue interface modules illustrated in Figs. 23-32 provide a seal (see for example, intermediate gasket 600) between tissue interface module 116 and applicator 114, thereby separating applicator chamber 118 from tissue acquisition chamber 142. This seal prevents air from flowing directly from tissue acquisition chamber 142 to applicator chamber 118, facilitating the flow of air through filter 154 and air passages 602 along air flow path A in Figs. 28 and 32 when a vacuum is applied to applicator chamber 118, through, for example, vacuum interface 504. In these embodiments of the invention, air extracted from applicator chamber 118 will flow along path B as illustrated in Figs. 28 and 32.

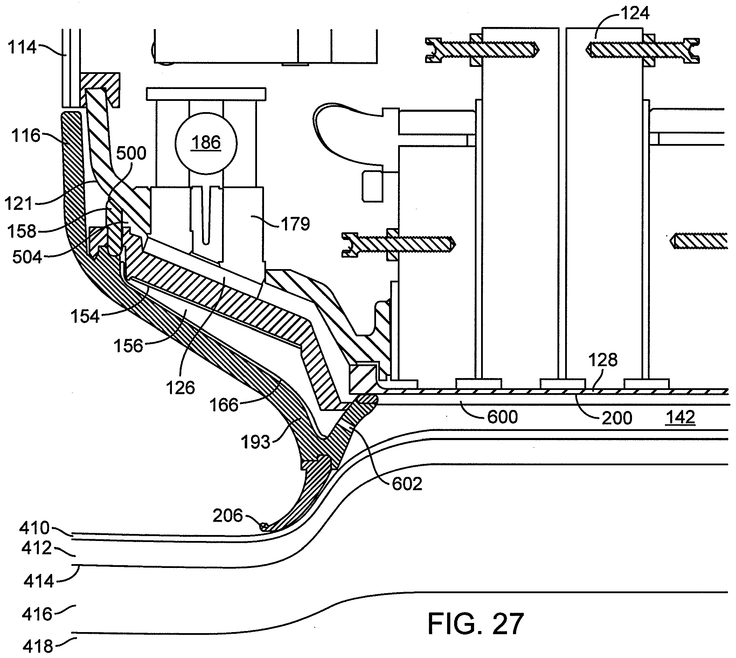

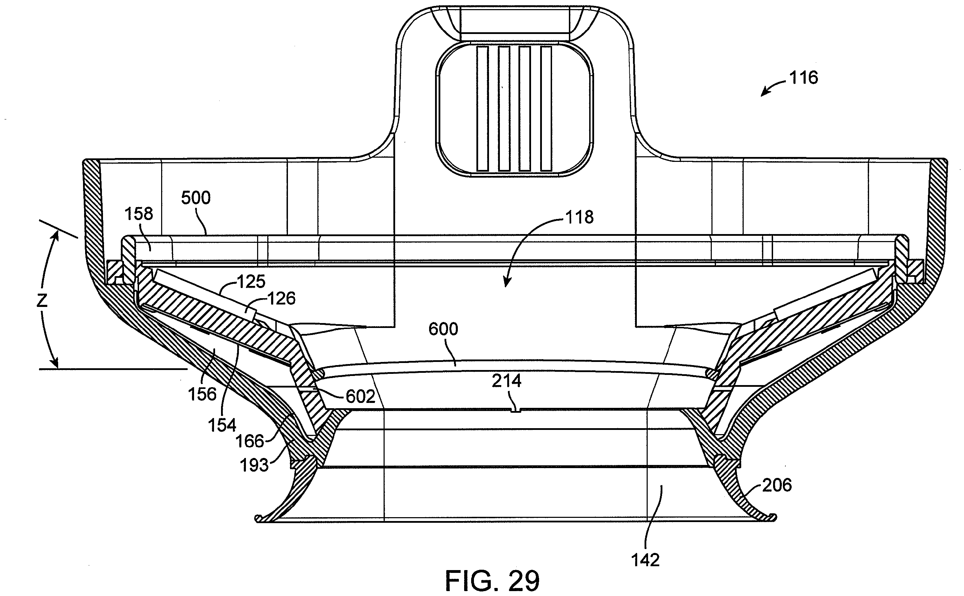

As shown in Figure 27, when the skirt 206 of tissue interface module 116 is placed against a patient's skin surface, the vacuum created in tissue acquisition chamber 142 in response to movement of air along these flow paths A and B will draw the patient's skin and underlying tissue (including epidermis 410, dermis 412, dermal-hypodermal interface 414, hypodermis 416 and muscle 418) into tissue acquisition chamber 142 toward the applicator cooling plate 128. The flow restricting nature of filters 154 may, in some embodiments be used to ensure that the air pressure in tissue acquisition chamber 142 is higher than the air pressure in applicator chamber 118 until the patient's tissue ceases moving into tissue acquisition chamber 142, at which point the air pressures in the two chambers will equalize. In embodiments of the tissue interface module illustrated in, for example, Figs. 23 through 28, intermediate seal, in the form of, for example, intermediate gasket 600 may be positioned such that, when attached to applicator 114 gasket 600 forms an air tight or substantially air tight seal against a surface of cooling plate 128. In embodiments of the tissue interface module 116 illustrated in, for example, Figs. 29 through 32, intermediate seal, in the form of, for example, intermediate gasket 600 may be positioned such that, when attached to applicator 114 gasket 600 is adapted to form an air tight or substantially air tight seal against an outer surface of applicator 114. In embodiments of the invention illustrated in, for example Figs. 29 - 32, air passages 602 may be positioned outside of tissue acquisition chamber 142 to reduce or eliminate the potential for air passages 602 to be blocked by tissue in tissue acquisition chamber 142. Some embodiments, such as, for example, those in Figure 29 may also include vacuum notches 214 to facilitate the flow of air from tissue acquisition chamber 142 around the distal end of applicator 114 (when tissue interface module 116 is positioned on applicator 114) and into air passages 602.

In the embodiments of the invention illustrated in Fig. 23, engagement surface 125 may form an Angle Y between engagement surface 125 and a plane passing through gasket 600. In the embodiments of the invention illustrated in Fig. 29, engagement surface 125 may form an Angle Z between engagement surface 125 and a plane passing through gasket 600. In other embodiments of the invention, Angles Y and Z may be measured between engagement surface 125 and a plane running through or parallel to the distal surface of cooling plate 128 when tissue interface module 116 is positioned on applicator 114. In embodiments of the invention, Angles Y and Z may be approximately 22.5 degrees. In embodiments of the invention, Angles Y and Z may be between approximately 17.5 degrees and 27.5 degrees, or alternatively, between approximately 12.5 degrees and 32.5 degrees. In other embodiments, Angles Y and Z may vary, up to and including 45 degrees or more, depending upon the angle chosen for the mating engagement surfaces on applicator 114.

In the embodiments of the invention illustrated in Figs. 23 through 32, air flows through tissue interface module 116 when a vacuum is applied to vacuum interface 504 at the proximal end of the tissue interface module 116. With tissue interface module 116 positioned on applicator 114 and tissue engaged, as, for example, in Fig. 27, air trapped in tissue acquisition chamber 142 may flow, through the air passage A, including air passages 602, fluid trap 156, through filter 154 and vacuum channels 138, under attachment mechanism 126, through applicator chamber 118 to vacuum interface 504 and into vacuum inlet 174 in applicator 114. In embodiments of the invention, such as, for example, the embodiments illustrated in Figs. 23-32, air may be prevented from bypassing filter 152 by the intermediate sealing member 600. In embodiments of the invention, such as, for example, the embodiments illustrated in Figs. 1 -22, air may be prevented from bypassing filter 152 by bio-barrier 152. Air in applicator chamber 118 flows along path B through vacuum interface 504 and into vacuum inlet 174.

One aspect of the invention provides a tissue interface module for use with an applicator in a microwave-based tissue modification system. The tissue interface module has an applicator chamber on a proximal side of the tissue interface module and a tissue acquisition chamber on a distal side of the tissue interface module. The applicator chamber may include: an opening adapted to receive the applicator; an attachment mechanism positioned in the applicator chamber and adapted to attach the tissue interface module to the applicator; a sealing member positioned at a proximal side of the applicator chamber; and a vacuum interface positioned at a proximal side of the applicator chamber and adapted to receive a vacuum inlet positioned on a distal end of the applicator. The tissue acquisition chamber may include a tissue acquisition opening on a distal side of the tissue interface module. The system may also include a flexible bio-barrier positioned between, and in fluid communication with, the applicator chamber and the tissue acquisition chamber, the flexible bio-barrier being substantially impermeable to air or fluids; an airflow pathway within the tissue interface module, the airflow pathway connecting the applicator chamber and the tissue acquisition chamber; and a filter disposed in the airflow pathway connecting the applicator chamber and the tissue acquisition chamber, the filter being permeable to air and substantially impermeable to fluids.

In some embodiments, the tissue interface module may also include a variable flow restrictor between, and in communication with, the tissue acquisition chamber and the filter. The variable flow restrictor may be positioned in the airflow pathway. The variable flow restrictor may be a flexible element adapted to expand a flow opening in the airflow pathway in response to a pressure difference between the tissue acquisition chamber and the filter.

In some embodiments, the sealing member forms at least a portion of the vacuum interface and is adapted to provide a substantially air tight seal against a sealing surface on the applicator when the tissue interface module is attached to the applicator with the attachment mechanism.

Another aspect of the invention provides a tissue interface module for use with an applicator in a microwave-based tissue modification system. The tissue interface module has an applicator chamber on a proximal side of the tissue interface module and a tissue acquisition chamber on a distal side of the tissue interface module. The applicator chamber may include: an opening adapted to receive an applicator; at least one attachment plate positioned in the applicator chamber, the attachment plate adapted to magnetically attach to elements of a magnetic circuit positioned on a distal end of the applicator; a sealing member positioned at a proximal side of the applicator chamber; a vacuum interface positioned at a proximal side of the applicator chamber and adapted to connect to a vacuum source. The tissue acquisition chamber may include a tissue acquisition opening on a distal side of the tissue interface module. The tissue interface module may also have a flexible bio-barrier positioned between, and in fluid communication with, the applicator chamber and the tissue acquisition chamber, the flexible bio-barrier being substantially impermeable to air or fluids; an airflow pathway within the tissue interface module, the airflow pathway connecting the applicator chamber and the tissue acquisition chamber; and a filter disposed in the airflow pathway connecting the applicator chamber and the tissue acquisition chamber, the filter being permeable to air and substantially impermeable to fluids.

In some embodiments, the attachment plate has a magnetic element adapted to form a magnetic circuit with magnetic elements in the applicator. The attachment plate may be, e.g., a ferromagnetic plate.

In some embodiments, the tissue interface module has a tissue interface module engagement surface adapted to engage with a corresponding applicator engagement surface on the applicator, the tissue interface module engagement surface being disposed at an angle of approximately 17.5 degrees to 27.5 degrees, such as approximately 22.5 degrees, with respect to the flexible bio-barrier. In some such embodiments, the attachment mechanism includes a ferromagnetic plate and the tissue interface module engagement surface includes a surface of the ferromagnetic plate.

Yet another aspect of the invention provides a tissue interface module for use with an applicator in a microwave-based tissue modification system. The tissue interface module has an applicator chamber on a proximal side of the tissue interface module and a tissue acquisition chamber on a distal side of the tissue interface module. The applicator chamber may include: an opening adapted to receive an applicator; an attachment mechanism positioned in the applicator chamber and adapted to attach the tissue interface module to the applicator; a sealing member positioned at a proximal side of the applicator chamber; and a vacuum interface positioned at the proximal side of the applicator chamber and adapted to connect to a vacuum source. The tissue acquisition chamber may have a tissue acquisition opening on a distal side of the tissue interface module. The tissue interface module may also have a flexible bio-barrier positioned between, and in fluid communication with, the applicator chamber and the tissue acquisition chamber, the flexible bio-barrier being substantially impermeable to air or fluids and may also be substantially transparent to microwave energy; a vacuum pathway within the tissue interface module, the vacuum pathway including an exit opening at a proximal end of the tissue acquisition chamber; and a filter disposed between the exit opening and the vacuum interface, the filter being permeable to air and substantially impermeable to fluids.

In some embodiments, the vacuum pathway extends from the distal end of the tissue acquisition chamber to the vacuum interface. The tissue interface module may also include a second filter disposed between, and communicating with, the applicator chamber and the tissue acquisition chamber, the second filter being permeable to air and substantially impermeable to fluids. In some such embodiments, the filter and the second filter are positioned on opposing sides of the bio-barrier. The functional surface area of the bio-barrier may also be approximately the same as the functional surface area of the filter and the second filter combined.

Still another aspect of the invention provides a tissue interface module having an applicator chamber on a proximal side and a tissue acquisition chamber on a distal side; a bio-barrier positioned between, and in fluid communication with, the applicator chamber and the tissue acquisition chamber, the bio-barrier being substantially impermeable, flexible, and microwave transparent; a vacuum path extending from a distal end of the tissue acquisition chamber to a proximal end of the applicator chamber and including a filter, a vacuum trap and an expandable aperture; the vacuum path being adapted to facilitate the flow of air from the tissue acquisition chamber, through the expandable aperture, through the vacuum trap, through the filter and into the applicator chamber when the applicator chamber is attached to a vacuum source.

In some embodiments, the tissue interface module also includes: an outer shell; an inner insert positioned in the outer shell to form a body of the tissue interface module; a gasket positioned on the inner insert and (i) providing a vacuum seal between the inner insert and the outer shell on a distal side of the gasket, (ii) being shaped to provide a vacuum seal to an applicator on a proximal side of the gasket, and (iii) forming a portion of the vacuum trap. The tissue interface module may also include a reflector (i) reflecting at least a portion of any microwave energy entering the applicator chamber; (ii) electrically isolated from an applicator positioned in the applicator chamber; (iii) positioned between the outer shell and the inner insert; and (iv) having a distal end surrounding at least a portion of the tissue acquisition chamber.

In some embodiments, the tissue interface module also has a latch plate positioned in the applicator chamber on the inner insert and including an attachment surface forming a predetermined angle with the bio-barrier when the bio-barrier is in a first position. The predetermined angle may be between 17.5 degrees and 27.5 degrees, such as approximately 22.5 degrees.

Yet another aspect of the invention provides a method of treating a patient including the following steps: attaching a tissue interface module to an applicator, wherein the distal end of the applicator is positioned in an applicator chamber of the tissue interface module; placing a distal opening of a tissue acquisition chamber of the tissue interface module against a tissue surface; pulling a portion of the patient's skin into the tissue acquisition chamber by creating a vacuum in the tissue acquisition chamber, the vacuum being created by drawing air from the tissue acquisition chamber to a vacuum source in the applicator through a vacuum path including the applicator chamber and a filter between the applicator chamber and the tissue acquisition chamber; and applying microwave energy to tissue positioned in the tissue acquisition chamber.

In some embodiments, the method also includes the step of cooling tissue in the tissue acquisition chamber during the application of microwave energy. In some embodiments, air is pulled from the tissue acquisition chamber, through the filter, into the applicator chamber and into a vacuum interface positioned on the distal end of the applicator.

In some embodiments, the applicator chamber and the tissue acquisition chamber are separated by, and in fluid communication with, a flexible bio-barrier. In such embodiments, the step of applying microwave energy can include the step of applying such energy through the flexible bio-barrier, and the step of pulling a portion of the patient's skin into the tissue acquisition chamber pulls the flexible bio-barrier against a distal end of the applicator.

In some embodiments, the method includes the step of varying a size of an opening between the tissue acquisition chamber and the filter during the step of creating a vacuum, such as by pulling the flexible bio-barrier against the distal end of the applicator.

Another aspect of the invention provides a tissue interface module for use with an applicator in a microwave-based tissue modification system, the tissue interface module including: an attachment mechanism on a proximal side of the tissue interface module adapted to attach to an applicator; an applicator chamber adapted to receive a microwave antenna, a cooling element, and a vacuum port of the applicator, the applicator chamber comprising a bio-barrier on a distal side; a tissue acquisition chamber having a tissue acquisition opening on a distal side of the tissue interface module; and a filter disposed between, and communicating with, the applicator chamber and the tissue acquisition chamber, the filter having openings configured to permit air to pass and to prevent liquid from passing.

In some embodiments, the tissue interface module also has a variable flow restrictor between, and in communication with, the tissue acquisition chamber and the filter. The variable flow restrictor may have a flexible element adapted to expand a flow opening between the tissue acquisition chamber and the filter in response to a pressure difference between the tissue acquisition chamber and the filter.

In some embodiments, the attachment mechanism has a magnetic element (such as, e.g., a ferromagnetic plate) adapted to magnetically attach to a corresponding element in the applicator. The tissue interface module may also have a tissue interface module engagement surface adapted to engage with a corresponding applicator engagement surface on the applicator, the tissue interface module engagement surface being disposed at an angle of approximately 12.5 degrees to 32.5 degrees, or approximately 17.5 degrees to 27.5 degrees, or approximately 22.5 degrees, with respect to the bio-barrier.

In some embodiments, the tissue interface module also has a vacuum flow path from the tissue acquisition chamber, through the filter, into the applicator chamber. In some embodiments, the tissue interface module has a vacuum flow path from the tissue acquisition chamber, through the filter, through the applicator chamber, into the vacuum port of the applicator.

In some embodiments, the tissue interface module may also have a second filter disposed between, and communicating with, the applicator chamber and the tissue acquisition chamber, the second filter having openings configured to permit air to pass and to prevent liquid from passing. In some such embodiments, the filter and the second filter are positioned on opposing sides of the bio-barrier. The bio-barrier may have approximately the same surface area as the filter and the second filter combined.

Another aspect of the invention provides a method of treating tissue of a patient including the following steps: attaching a tissue interface module to an applicator to place a microwave antenna, a cooling plate, and a vacuum port within an applicator chamber of the tissue interface module; placing a distal opening of a tissue acquisition chamber of the tissue interface module against a tissue surface; drawing a vacuum from a vacuum source in the applicator through the applicator chamber, a filter between the applicator chamber and the tissue acquisition chamber; and applying microwave energy to the patient's tissue.

Some embodiments add the step of varying a size of an opening between the tissue acquisition chamber and the filter during the step of drawing a vacuum, such as by moving a flexible member to change the size of the opening. In some embodiments, the attaching step includes the step of magnetically coupling the tissue interface module to the applicator.

In some embodiments, the applicator chamber has a bio-barrier on a distal side, and the attaching step includes the step of engaging a magnetic tissue interface module engagement surface with a corresponding applicator engagement surface on the applicator, the tissue interface module engagement surface being disposed at an angle of approximately 17.5 degrees to 27.5 degrees with respect to the bio-barrier.

Still another aspect of the invention provides a microwave-based tissue modification system having: a microwave applicator with a microwave antenna, a cooling element, and a vacuum port; and a tissue interface module with: an attachment mechanism on a proximal side of the tissue interface module adapted to attach to the microwave applicator; an applicator chamber adapted to connect to the microwave antenna, the cooling element, and the vacuum port of the microwave applicator, the applicator chamber having a bio-barrier on a distal side; a tissue acquisition chamber having a tissue acquisition opening on a distal side of the tissue interface module; and a filter disposed between, and communicating with, the applicator chamber and the tissue acquisition chamber, the filter having openings configured to permit air to pass and to prevent liquid from passing.

In some embodiments, the tissue modification system also has a variable flow restrictor between, and in communication with, the tissue acquisition chamber and the filter. In some embodiments, the attachment mechanism includes a magnetic element adapted to magnetically attach to a corresponding element in the applicator. The tissue modification system may also include a tissue interface module engagement surface adapted to engage with a corresponding applicator engagement surface on the microwave applicator, the tissue interface module engagement surface being disposed at an angle of approximately 17.5 degrees to 27.5 degrees with respect to the bio-barrier.

In some embodiments, the tissue modification system has a vacuum flow path from the tissue acquisition chamber, through the filter, through the applicator chamber, into the vacuum port of the microwave applicator. The tissue interface module may also have a second filter disposed between, and communicating with, the applicator chamber and the tissue acquisition chamber, the second filter having openings configured to permit air to pass and to prevent liquid from passing. In some such embodiments, the filter and the second filter are positioned on opposing sides of the bio-barrier.

Yet another aspect of the invention provides a tissue interface module for use with an applicator in a microwave-based tissue modification system, the tissue interface module having: an attachment mechanism on a proximal side of the tissue interface module adapted to attach to an applicator, the attachment mechanism including an engagement surface that forms an angle of approximately 17.5 degrees to 27.5 degrees from horizontal; an applicator chamber adapted to connect to a microwave antenna, a cooling element, and a vacuum port of the applicator, the applicator chamber comprising a bio-barrier on a distal side, wherein the bio-barrier is configured to prevent air and liquid from passing; a tissue acquisition chamber having a tissue acquisition opening defined by a skirt on a distal side of the tissue interface module; and a filter disposed between, and communicating with, the applicator chamber and the tissue acquisition chamber, the filter having openings configured to permit air to pass and to prevent liquid from passing.

In some embodiments, the tissue interface module has a vacuum flow path from the tissue acquisition chamber, through the filter, through the applicator chamber, into the vacuum port of the microwave applicator. The tissue interface module may also include a second filter disposed between, and communicating with, the applicator chamber and the tissue acquisition chamber, the second filter having openings configured to permit air to pass and to prevent liquid from passing. In some such embodiments, the filter and the second filter are positioned on opposing sides of the bio-barrier. In some embodiments, the tissue interface module also has a fluid trap disposed between the tissue acquisition chamber and the filter, the fluid trap configured to capture tissue and liquid.

Another aspect of the invention provides a consumable medical device having: an applicator chamber positioned on a proximal side of said consumable; a tissue chamber positioned on a distal side of said consumable medical device; a first bio-barrier positioned between the applicator chamber and the tissue chamber, the first bio-barrier being: substantially impermeable, flexible and microwave transparent; a vacuum path extending from a distal end of the applicator chamber to a proximal end of the tissue chamber and including a second bio-barrier, a vacuum trap, and an expandable aperture, the vacuum path being adapted to facilitate the flow of air from the tissue chamber, through the expandable aperture, through the vacuum trap, through the second bio-barrier and into the applicator chamber.

In some embodiments, the consumable also includes a shell; an insert positioned in the shell to form a body of said consumable; a gasket positioned on the insert, providing a vacuum seal between the insert and the shell on a distal side of the gasket, being shaped to provide a vacuum seal to an applicator on a proximal side of the gasket and forming a portion of the vacuum trap.

In some embodiments, the consumable also includes a reflector reflecting at least a portion of any microwave energy entering the applicator chamber, the reflector being electrically isolated from an applicator positioned in said applicator chamber, being positioned between the shell and the insert, and having a distal end surrounding at least a portion of the tissue chamber.

In some embodiments, the consumable also has a latch plate positioned in the applicator chamber on said insert, forming a predetermined angle with the first bio-barrier when the first bio-barrier is in a first position.

Still another aspect of the invention provides a method of transmitting energy to a patient for the purpose of reducing sweat, the method including the steps of: transmitting the energy through an applicator having: an antenna; a field spreader, a fluid channel, and a cooling plate; and transmitting the energy through a consumable having: an applicator chamber; a flexible bio-barrier; and a tissue chamber.

Yet another aspect of the invention provides a consumable including a flexible bio-barrier and a cooling plate configured to cooperate to form expandable channel connecting a tissue chamber to an applicator chamber, the consumable including a vacuum path wherein air from a the tissue chamber passes through: the expandable channel; a fluid trap; a second bio-barrier; vacuum channels separating second bio-barrier from an attachment mechanism (such as, e.g., a magnetic plate); and an applicator chamber.

Another aspect of the invention provides a multifunctional connector adapted to connect an applicator to a microwave generator console through a cable assembly, the connector having: a cooling fluid connector; a cooling fluid return connector; a microwave connector; electronic connectors; and vacuum connectors.

As for additional details pertinent to the present invention, materials and manufacturing techniques may be employed as within the level of those with skill in the relevant art. The same may hold true with respect to method-based aspects of the invention in terms of additional acts commonly or logically employed. Also, it is contemplated that any optional feature of the inventive variations described may be set forth and claimed independently, or in combination with any one or more of the features described herein. Likewise, reference to a singular item, includes the possibility that there are plural of the same items present. More specifically, as used herein and in the appended claims, the singular forms "a," "and," "said," and "the" include plural referents unless the context clearly dictates otherwise. The invention is defined in the claims as follows.

## Claims

1. A tissue interface module (116) for use with an applicator in a microwaved-based tissue modification system, comprising:
an applicator chamber (118), said applicator chamber being positioned on a proximal side of said tissue interface module and being configured to be coupled to an applicator having a vacuum port;
a tissue acquisition chamber (142), said tissue acquisition chamber being positioned on a distal side of said tissue interface module;
a central opening between the applicator chamber to the tissue acquisition chamber; an intermediate sealing member (600) surrounding at least a portion of the central opening and adapted to provide a seal between the tissue interface module and the applicator and to prevent air flow through the central opening;
a vacuum path within the tissue interface module (116), said vacuum path extending from a proximal side of the intermediate sealing member (600) to a distal side of the intermediate sealing member (600) and bypassing the intermediate sealing member and the central opening, the vacuum path comprising a filter (154) and a vacuum trap;
wherein the filter (154) is configured to permit air to pass and to prevent liquid from passing; and
wherein the vacuum path is adapted to facilitate the flow of air from the tissue acquisition chamber through the vacuum trap, from the vacuum trap through the filter, and from the filter into the applicator chamber, when the applicator chamber is coupled to the vacuum port of the applicator.

2. The tissue interface module (116) of claim 1 wherein the tissue interface module comprises a vacuum interface (504) that is adapted to receive a vacuum inlet positioned on a distal end of the applicator.

3. The tissue interface module (116) of claim 1 further including a bio-barrier positioned between, and in fluid communication with, said applicator chamber (118) and said tissue acquisition chamber (142).

4. The tissue interface module (116) of claim 3 wherein the bio-barrier is substantially impermeable, flexible and microwave transparent.

5. The tissue interface module (116) of claim 1 further including an attachment plate positioned in the applicator chamber.

6. The tissue interface module (116) of claim 5 wherein the attachment plate comprises a magnet.

## Patentansprüche

1. Gewebeschnittstellenmodul (116) zur Verwendung mit einem Applikator in einem System zur mikrowellenbasierten Gewebemodifikation, umfassend:
eine Applikatorkammer (118), wobei die Applikatorkammer auf einer proximalen Seite des Gewebeschnittstellenmoduls positioniert ist und konfiguriert ist, um an einen Applikator gekoppelt zu werden, der einen Vakuumöffnung aufweist;
eine Gewebeaufnahmekammer (142), wobei die Gewebeaufnahmekammer auf einer distalen Seite des Gewebeschnittstellenmoduls positioniert ist;
eine zentrale Öffnung zwischen der Applikatorkammer und der Gewebeaufnahmekammer;
ein Zwischendichtelement (600), das mindestens einen Abschnitt der zentralen Öffnung umschließt und ausgebildet ist, um eine Abdichtung zwischen dem Gewebeschnittstellenmodul und dem Applikator bereitzustellen und um eine Luftströmung durch die zentrale Öffnung zu verhindern;
einen Vakuumpfad innerhalb des Gewebeschnittstellenmoduls (116), wobei sich der Vakuumpfad von einer proximalen Seite des Zwischendichtelements (600) zu einer distalen Seite des Zwischendichtelements (600) erstreckt und das Zwischendichtelement und die zentrale Öffnung umgeht, wobei der Vakuumpfad einen Filter (154) und eine Vakuumfalle umfasst;
wobei der Filter (154) konfiguriert ist, um Luft durchströmen zu lassen und Flüssigkeit am Durchfließen zu hindern; und
wobei der Vakuumpfad ausgebildet ist, um Luftströmung von der Gewebeaufnahmekammer durch die Vakuumfalle, von der Vakuumfalle durch den Filter und von dem Filter in die Applikatorkammer zu erleichtern, wenn die Applikatorkammer an den Vakuumöffnung des Applikators gekoppelt ist.

2. Gewebeschnittstellenmodul (116) nach Anspruch 1, wobei das Gewebeschnittstellenmodul eine Vakuumschnittstelle (504) umfasst, die ausgebildet ist, um einen an einem distalen Ende des Applikators positionierten Vakuumeingang zu empfangen.

3. Gewebeschnittstellenmodul (116) nach Anspruch 1, ferner einschließend eine Biobarriere, die zwischen, und in Fluidkommunikation mit, der Applikatorkammer (118) und der Gewebeaufnahmekammer (142) positioniert ist.

4. Gewebeschnittstellenmodul (116) nach Anspruch 3, wobei die Biobarriere im Wesentlichen undurchlässig, elastisch und mikrowellentransparent ist.

5. Gewebeschnittstellenmodul (116) nach Anspruch 1, ferner einschließend eine Befestigungsplatte, die in der Applikatorkammer positioniert ist.

6. Gewebeschnittstellenmodul (116) nach Anspruch 5, wobei die Befestigungsplatte einen Magneten umfasst.

## Revendications

1. Module d'interface tissulaire (116) destiné à être utilisé avec un applicateur dans un système de modification de tissus à base de microondes, comprenant :
une chambre de l'applicateur (118), ladite chambre de l'applicateur étant positionnée sur un côté proximal dudit module d'interface tissulaire et étant configurée pour être couplé à un applicateur comportant un orifice d'aspiration ;
une chambre d'acquisition de tissus (142), ladite chambre d'acquisition des tissus étant positionnée sur un côté distal dudit module d'interface tissulaire ;
une ouverture centrale entre la chambre de l'applicateur et la chambre d'acquisition tissulaire ;
un élément d'étanchéité intermédiaire (600) entourant au moins une partie de l'ouverture centrale et adapté pour établir un joint d'étanchéité entre le module d'interface tissulaire et l'applicateur et pour empêcher un écoulement d'air à travers l'ouverture centrale ;
un passage de vide dans le module d'interface tissulaire (116), ledit trajet sous vide s'étendant d'un côté proximal de l'élément d'étanchéité intermédiaire (600) à un côté distal de l'élément d'étanchéité intermédiaire (600) et contournant l'élément d'étanchéité intermédiaire et l'ouverture centrale, le passage de vide comprenant un filtre (154) et un piège à vide;
dans lequel le filtre (154) est configuré pour laisser passer l'air et empêcher le liquide de passer; et
dans lequel le passage de vide est adapté pour faciliter la circulation de l'air de la chambre d'acquisition tissulaire à travers le piège à vide, du piège à vide à travers le filtre, et du filtre dans la chambre de l'applicateur, lorsque la chambre de l'applicateur est couplée à l'orifice de vide de l'applicateur.

2. Module d'interface tissulaire (116) selon la revendication 1, dans lequel le module d'interface tissulaire comprend une interface sous vide (504) adaptée pour recevoir une entrée de vide positionnée sur une extrémité distale de l'applicateur.

3. Module d'interface tissulaire (116) selon la revendication 1, incluant en outre une barrière bio positionnée entre et en communication fluidique avec ladite chambre d'applicateur (118) et ladite chambre d'acquisition de tissu (142).

4. Module d'interface tissulaire (116) selon la revendication 3, dans lequel la barrière bio est substantiellement imperméable, flexible et transparente aux micro-ondes.

5. Module d'interface tissulaire (116) selon la revendication 1, incluant en outre une plaque de fixation positionnée dans la chambre de l'applicateur.

6. Module d'interface tissulaire (116) selon la revendication 5, dans lequel la plaque de fixation comprend un aimant.
